# EUROPEAN PATENT APPLICATION

(11) **EP 4 763 204 A1**
(43) Date of publication of application: **24.06.2026**
(21) Application number: 24222958.1
(22) Date of filing: 23.12.2024
(51) Int. Cl.: A61K 31/26, A61K 9/00, A61K 45/06, A61P 7/02

(54) **ISOTHIOCYANATES FOR USE IN THE TREATMENT AND/OR PREVENTION OF A THROMBOTIC DISORDER**

(71) Applicant: Technische Universität München, in Vertretung des Freistaats Bayern, 80333 München (DE)
(72) Inventor: Wollenberg, Barbara, 80689 München (DE)
(74) Representative: Engelhard, Markus

(57) **Abstract**

The present invention relates to compositions comprising isothiocyanate for use in a method of treatment and/or prevention of a thrombotic disorder. In particular, the present invention relates to a composition comprising isothiocyanate for use in a method of treatment and/or prevention of a thrombotic disorder, wherein the isothiocyanate is selected from 2-phenylethyl-isothiocyanate (PEITC), benzyl-isothiocyanate (BITC), and/or combinations of PEITC and BITC, wherein, optionally, the composition further comprises one or more further isothiocyanate(s), such as allyl-isothiocyanate (AITC). Moreover, the present invention relates to kits and portable medical injection devices comprising said composition. Moreover, the present invention relates to a method of treatment comprising administering said composition to a patient in need thereof.

## Description

### FIELD OF THE INVENTION

The present invention relates to the general field of thrombotic disorders and treatment thereof. In particular, the present invention relates to compositions comprising isothiocyanate for use in a method of treatment and/or prevention of a thrombotic disorder. Moreover, the present invention relates to kits and portable medical injection devices comprising said composition.

### BACKGROUND OF THE INVENTION

Thrombotic disorders encompass a range of conditions characterized by the abnormal formation of blood clots (thrombi) within the blood vessels, which can obstruct the flow of blood through the circulatory system. These disorders can be broadly categorized into arterial and venous thrombosis, leading to a range of serious health conditions. Arterial thrombosis can result in acute events such as myocardial infarction (heart attack) and ischemic stroke, while venous thrombosis can lead to conditions such as deep vein thrombosis and pulmonary embolism. The consequences of thrombosis can be life-threatening, making it a critical focus of medical research and treatment.

Thrombotic disorders, such as thrombosis, are a significant global health issue, with millions of cases reported annually. Venous thromboembolism, which includes deep vein thrombosis and pulmonary embolism, affects approximately 1 to 2 per 1,000 people each year. The incidence of arterial thrombosis, particularly myocardial infarction and ischemic stroke, is also high, contributing to substantial morbidity and mortality worldwide. The aging population, increasing prevalence of risk factors such as obesity, sedentary lifestyle, and chronic diseases like diabetes and hypertension, further exacerbate the incidence of thrombotic events.

Platelet aggregation plays a crucial role in the pathophysiology of thrombosis, particularly in arterial thrombosis. Platelets (PLTs) are anucleated cells derived from megakaryocytes. For a long time, it was considered that the main function of PLTs was to reduce blood loss upon injury. However, it is now apparent that in addition to hemostasis, thrombus formation and atherosclerosis, PLTs also play a role in inflammation and the immune response. Under physiological conditions, PLT activation is prevented by prostaglandin I₂ (PGI₂) and NO, which are secreted from the vascular endothelium. Upon injury or in other pathological settings, PLTs can be activated by binding of collagen, fibrinogen, thrombin, the von Willebrand Factor (vWF), ADP, thromboxane A₂ (TXA₂), immunoglobulin G antibodies (IgGs) or immune complexes to receptors on the PLT surface. PLT activation leads to the initiation of signaling cascades that result in PLT shape change and degranulation, which promotes further PLT activation. Furthermore, PLT activation leads to expression of P-Selectin (CD62P), CD63 and CD40L at the PLT surface, the first two being markers of PLT degranulation. PLT activation also results in enhanced ROS levels, which further activate PLT, whereas too high ROS levels inhibit PLT activation. Ultimately, PLT activation results in PLT aggregation, which is needed for vascular wound closure, however, is detrimental, when no wound is present and a thrombus is built instead. Therefore, when PLT function is altered, risk for thrombosis is increased with potentially lethal outcomes in the form of stroke, myocardial infarction or pulmonary embolism.

Already in the 1950s it was found that plants release essential oils containing isothiocyanates (ITCs) upon destruction of plant cells. These ITCs develop antibacterial effects, through which especially cruciferous plants fight off pathogens, such as bacteria or fungi. In plants, ITCs are synthesized as glucosinolate precursors and are processed by plant myrosinase upon plant injury. Antibacterial and antifungal activity is known for several ITCs. The immunomodulatory activity of ITCs is also used in medicinal products comprising benzyl-isothiocyanate (BITC), allyl-isothiocyanate (AITC) and 2-phenylethyl-isothiocyanate (PEITC) derived from *Tropaeoli majoris herba* (nasturtium) and *Armoraciae rusticanae radix* (horseradish). These products are applied as prescription-free, preventive phytotherapeutics for acute sinusitis, acute bronchitis, or for prophylactic treatment of patients with chronically recurrent lower urinary tract infections.

Previous studies also addressed the immunomodulatory effects of ITCs and beneficial effects of ITCs were also observed in several cancers, where ITCs reduced migration, proliferation and invasion. ITCs also showed anticancer-activity by enhancing susceptibility to chemotherapy, and enhancing apoptosis.

Concerning thrombotic disorders, currently several anti-thrombotic drugs are used in the clinics to reduce risk of thrombosis, such as COX inhibitors (e.g., aspirin), vitamin K antagonists or thrombin inhibitors. However, only plasminogen activators are thrombolytic. Since plasminogen activators severely increase bleeding risk, new approaches to prevent and reverse PLT aggregation are needed.

The limitations of current treatment options highlight the need for novel means for the treatment and/or prevention of thrombotic disorders. Moreover, there is a need for medicine devices and/or kits comprising the novel means for the treatment and/or prevention of thrombotic disorders.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a composition comprising isothiocyanate for use in a method of treatment and/or prevention of a thrombotic disorder, wherein the isothiocyanate is selected from 2-phenylethyl-isothiocyanate (PEITC), benzyl-isothiocyanate (BITC), and/or combinations of PEITC and BITC, wherein, optionally, the composition for use further comprises one or more further isothiocyanate(s).

In one embodiment, the one or more further isothiocyanate(s) is (are) selected from a group comprising allyl-isothiocyanate (AITC), sulforaphane, and isopropyl isothiocyanate; wherein, preferably, the one or more further isothiocyanate(s) is (are) AITC.

In one embodiment, the composition for use comprises as isothiocyanate:
PEITC;
BITC;
a combination of PEITC and BITC;
a combination of PEITC and AITC;
a combination of BITC and AITC; or
a combination of PEITC, BITC, and AITC;

wherein, preferably, the composition for use comprises:
   PEITC;
   a combination of PEITC and BITC;
   a combination of PEITC and AITC;
   a combination of BITC and AITC; or
   a combination of PEITC, BITC and AITC;
wherein, more preferably, the composition for use comprises:
   a combination of PEITC and BITC;
   a combination of PEITC and AITC; or
   a combination of PEITC, BITC and AITC;
wherein, even more preferably the composition for use comprises a combination of PEITC, BITC and AITC.

In one embodiment, if the composition for use comprises a combination of PEITC, BITC and AITC, the mass ratio of PEITC : AITC : BITC is in the range from about 100 : 10 : 1 to about 1 : 10 : 100, preferably in the range from about 50 : 5 : 1 to about 1 : 5 : 50, even more preferably in the range from about 10 : 5 : 1 to about 1 : 5 : 10, most preferably the mass ratio is about 1 : 3.1 : 4.1.

In one embodiment, if the composition for use comprises a combination of PEITC, BITC and AITC, the mass ratio of PEITC : AITC : BITC is in the range from about 100 : 10 : 1 to about 1 : 10 : 100, preferably in the range from about 50 : 5 : 1 to about 1 : 10 : 50, even more preferably in the range from about 10 : 5 : 1 to about 1 : 8 : 10, even more preferably in the range from about 5 : 5 : 1 to about 1 : 8 : 10, even more preferably in a range of about 1 : 1 : 1 to about 1 : 8 : 10, even more preferably in a range of about 1 : 1.5 : 2 to about 1 : 6 : 8, even more preferably in a range of about 1 : 2 : 3 to about 1 : 4 : 6, even more preferably in a range of about 1 : 2.5 : 3.5 to about 1 : 4 : 5, even more preferably the mass ratio is about 1 : 3.1 : 4.1, most preferably about 1 : 3.13 : 4.13.

In one embodiment, if the composition for use comprises a combination of PEITC, BITC and AITC, the mass ratio of PEITC : AITC : BITC is in the range from about 1 : 50 : 50 to about 50 : 50 : 1, preferably in the range from about 1 : 30 : 30 to about 30 : 30 : 1, even more preferably in the range from about 1 : 10 : 13 to about 10 : 1 : 1.3, even more preferably in the range from about 1 : 5 : 6.5 to about 5 : 1 : 1.3, even more preferably in a range of about 1 : 4 : 5.2 to about 4 : 1 : 1.3, even more preferably in a range of about 1 : 3.5 : 4.5 to about 3.5 : 1 : 1.3. Most preferably the mass ratio is about 1 : 3.1 : 4.1.

In one embodiment, if the composition for use comprises a combination of PEITC, BITC and AITC, the mass ratio of PEITC : AITC : BITC is about 1 : 3.1 : 4.1.

In one embodiment, if the composition for use comprises a combination of PEITC, BITC and AITC, the mass ratio of PEITC : AITC : BITC is about 1 : 3.13 : 4.13.

In one embodiment, the composition for use comprises a combination of PEITC, BITC and AITC, with about 37.9% AITC, 50% BITC and 12.1% PEITC.

In one embodiment, if the composition for use comprises a combination of PEITC and BITC, the mass ratio of PEITC : BITC is in the range from about 1 : 50 to about 50 : 1, preferably in the range from about 1 : 10 to about 10 : 1, even more preferably in the range from about 1 : 5 to about 5 : 1. Most preferably, the mass ratio of PEITC : BITC is about 4.1 : 1.

In one embodiment, if the composition for use comprises a combination of PEITC and BITC, the mass ratio of PEITC : BITC is in the range from about 1 : 50 to about 50 : 1, preferably in the range from about 1 : 30 to about 30 : 1, even more preferably in the range from about 1 : 20 to about 20 : 1, even more preferably in the range from about 1 : 15 to about 15 : 1, even more preferably in the range from about 1 : 10 to about 15 : 1, even more preferably in the range from about 1 : 5 to about 15 : 1, even more preferably in the range from about 1 : 2 to about 10 : 1, even more preferably in the range from about 1 : 1 to about 9 : 1, even more preferably in the range from about 2:1 to about 8.5 : 1, even more preferably in the range from about 2.05 : 1 to about 8.2 : 1, even more preferably in the range from about 3 : 1 to about 5 : 1. Most preferably, the mass ratio of PEITC : BITC is about 4.1 : 1.

In one embodiment, if the composition for use comprises a combination of PEITC and AITC, the mass ratio of PEITC : AITC is in the range from about 1 : 50 to about 50 : 1, preferably in the range from about 1 : 10 to about 10 : 1, even more preferably in the range from about 1 : 5 to about 5 : 1. Most preferably, the mass ratio of PEITC : AITC is about 3.1 : 1.

In one embodiment, if the composition for use comprises a combination of PEITC and AITC, the mass ratio of PEITC : AITC is in the range from about 1 : 50 to about 50 : 1, preferably in the range from about 1 : 30 to about 30 : 1, even more preferably in the range from about 1 : 20 to about 20 : 1, even more preferably in the range from about 1 : 15 to about 15 : 1, even more preferably in the range from about 1 : 10 to about 15 : 1, even more preferably in the range from about 1 : 5 to about 15 : 1, even more preferably in the range from about 1 : 2 to about 10 : 1, even more preferably in the range from about 1 : 1 to about 9 : 1, even more preferably in the range from about 1.55 : 1 to about 6.2 : 1, even more preferably in the range from about 2 : 1 to about 4 : 1. Most preferably, the mass ratio of PEITC : AITC is about 3.1 : 1.

In one embodiment, if the composition for use comprises a combination of PEITC and AITC, the mass ratio of PEITC : AITC is in the range from about 1 : 5 to about 5 : 1, even more preferably in the range from about 1 : 4 to about 4 : 1, even more preferably in the range from about 1 : 3.5 to about 3.5 : 1, even more preferably in the range from about 1 : 3.25 to about 3.25 : 1.

In one embodiment, if the composition for use comprises a combination of BITC and AITC, the mass ratio of BITC : AITC is in the range from about 1 : 50 to about 50 : 1, preferably in the range from about 1 : 10 to about 10 : 1, even more preferably in the range from about 1 : 5 to about 5 : 1. Most preferably, the mass ratio of BITC : AITC is about 1 : 1.3.

In one embodiment, if the composition for use comprises a combination of BITC and AITC, the mass ratio of BITC : AITC is in the range from about 1 : 50 to about 50 : 1, preferably in the range from about 1 : 30 to about 30 : 1, even more preferably in the range from about 1 : 20 to about 20 : 1, even more preferably in the range from about 1 : 10 to about 10 : 1, even more preferably in the range from about 1 : 5 to about 5 : 1, even more preferably in the range from about 1 : 4 to about 2 : 1, even more preferably in the range from about 1 : 2 to about 1 : 1. Most preferably, the mass ratio of BITC : AITC is about 1 : 1.3.

In one embodiment, if the composition for use comprises a combination of BITC and AITC, the mass ratio of BITC: AITC is in the range from about 1 : 5 to about 5 : 1, even more preferably in the range from about 1 : 4 to about 4 : 1, even more preferably in the range from about 1 : 3 to about 3 : 1, even more preferably in the range from about 1 : 2 to about 2 : 1, even more preferably in the range from about 1 : 1.5 to about 1.5 : 1, even more preferably in the range from about 1 : 1.35 to about 1.35 : 1.

In one embodiment, the thrombotic disorder is caused by one or more thrombi and/or one or more emboli.

In one embodiment, the thrombotic disorder is selected from a group comprising venous thrombosis or arterial thrombosis.

In one embodiment, the venous thrombosis is selected from a group comprising deep vein thrombosis, portal vein thrombosis, renal vein thrombosis, jugular vein thrombosis, Budd-Chiari syndrome, Paget-Schroetter disease, cerebral venous sinus thrombosis, mesenterial venous thrombosis.

In one embodiment, the arterial thrombosis is selected from a group comprising thrombotic stroke, myocardial infarction, pulmonary embolism, mesenterial infarction and acute arterial limb occlusion.

In one embodiment, the cause of the thrombotic disorder includes an activation and/or degranulation and/or aggregation of platelets.

In one embodiment, the thrombotic disorder affects an organ, preferably an organ selected from a group comprising the brain, the heart, the lung, the mesenterium, the kidney, and the liver.

In one embodiment, the composition for use is capable of preventing platelet activation, platelet degranulation and/or platelet aggregation and/or wherein the composition for use is capable of reversing platelet aggregation and/or platelet activation, preferably at the site of a thrombus or embolus.

In one embodiment, the composition for use is capable of acting as an anti-coagulant, preferably as a platelet-active drug.

In one embodiment, the composition for use is capable of lysing one or more thrombi and/or an emboli.

In one embodiment, the treatment and/or prevention involves the thrombolysis of one or more thrombi and/or emboli.

In one embodiment, the composition for use is used for thrombolysis.

In one embodiment, the composition for use is used in hemodialysis.

In one embodiment, the composition for use is used to prevent coagulation in blood samples.

In one embodiment, the composition for use is an anti-coagulant.

In one embodiment, the composition for use is a platelet-active drug.

In one embodiment, the composition for use is a pharmaceutical composition.

In one embodiment, the pharmaceutical composition comprises one or more further drug(s) or agent(s), such as pharmaceutically acceptable excipient(s) and/or carrier(s).

In one embodiment, the composition for use is administered to a patient in need thereof.

In one embodiment, the composition for use is administered to a patient in need thereof in a therapeutically effective amount.

In one embodiment, the composition for use is administered to a patient in need thereof in an amount sufficient to prevent platelet activation, platelet degranulation and/or platelet aggregation and/or in an amount sufficient to reverse platelet aggregation and/or platelet activation, preferably at the site of a thrombus or embolus.

In one embodiment, the composition for use is administered to a patient in need thereof in an amount sufficient to induce thrombolysis.

In one preferred embodiment, the patient is a human or an animal, preferably a human.

In one preferred embodiment, the patient is a human suffering from a thrombotic disorder.

In one preferred embodiment, the patient is a human that has been suffering from a thrombotic disorder.

In one embodiment, the patient is a human with a high predisposition for thrombotic disorders, such as a human exposed to risk factors, including genetic predispositions, acquired conditions (such as antiphospholipid syndrome), lifestyle factors (such as smoking, obesity, and prolonged immobility), and underlying medical conditions (such as cancer, diabetes, and cardiovascular diseases).

In one embodiment, the patient is a human who already suffered from one or more thrombotic disorders.

In one embodiment, the composition for use is administered via a route of administration selected from a group comprising:
a parenteral route of administration, such as intravenous administration, administration via a catheter, subcutaneous administration, intraperitoneal administration, intramuscular administration, intraarterial administration, nasal administration, intra-articular administration, intracardiac administration, intradermal administration, intralesional administration, intraocular administration, intraosseous administration, intrathecal administration, intravaginal administration, intracavernous administration, intravesical administration, intravitreal administration, transdermal administration, transmucosal administration, and perivascular administration;
an enteral route of administration, such as oral administration, sublingual administration, buccal administration, and rectal administration;
wherein, preferably, the composition for use is administered via a route of administration selected from a group comprising intravenous administration, administration via a catheter, subcutaneous administration, intraperitoneal administration, and oral administration;
more preferably selected from intravenous administration, administration via a catheter, subcutaneous administration, and intraperitoneal administration;
even more preferably selected from intravenous administration, administration via a catheter, and subcutaneous administration;
most preferably the composition for use is administered via intravenous administration.

In one embodiment, the composition for use is formulated for a route of administration selected from a group comprising:
a parenteral route of administration, such as intravenous administration, administration via a catheter, subcutaneous administration, intraperitoneal administration, intramuscular administration, intraarterial administration, nasal administration, intra-articular administration, intracardiac administration, intradermal administration, intralesional administration, intraocular administration, intraosseous administration, intrathecal administration, intravaginal administration, intracavernous administration, intravesical administration, intravitreal administration, transdermal administration, transmucosal administration, and perivascular administration;
an enteral route of administration, such as oral administration, sublingual administration, buccal administration, and rectal administration;
wherein, preferably, the composition for use is formulated for a route of administration selected from a group comprising intravenous administration, administration via a catheter, subcutaneous administration, intraperitoneal administration, and oral administration;
more preferably selected from intravenous administration, administration via a catheter, subcutaneous administration, and intraperitoneal administration;
even more preferably selected from intravenous administration and subcutaneous administration.

In one preferred embodiment, the composition for use is formulated for an intravenous administration.

In one embodiment, the composition for use is administered systematically, such as for systemic thrombolysis, or locally, such as for local thrombolysis.

In one preferred embodiment, systemic administration is performed via intravenous administration.

In one preferred embodiment, local administration is performed via a catheter (e.g. catheter lysis).

In one embodiment, the composition for use is administered for a treatment duration ranging from about 1 day to lifetime, preferably about 1 day to about 30 years, more preferably from about 2 days to about 10 years, even more preferably from about 2 days to about 2 years, even more preferably from about 3 days to about 18 months, even more preferably from about 1 week to about 1 year, most preferably from about 1 month to about 1 year.

In one embodiment, the administration is performed either once or the administration can be repeated.

In one embodiment, the treatment is continuous or intermittent.

In one embodiment, the composition for use is administered in conjunction with further agent(s) or drug(s), such as one or more further anticoagulant(s).

In one preferred embodiment, the one or more further anticoagulant(s) do not comprise active agents that contain a moiety that can be bound by ITCs.

In one preferred embodiment, the one or more further anticoagulant(s) do not comprise active agents that contain a thiol group and/or a amino group.

In one embodiment, the one or more further anticoagulant(s) are selected from the group comprising Heparin, streptokinase, urokinase, alteplase, reteplase, tenekteplase, GPIIb/IIIa antagonists such as abciximap or tirofiban, COX inhibitors such as aspirin or other non-steroidal anti-inflammatory drugs, and P2Y₁₂ antagonists such as clopidogrel.

In one embodiment, the composition for use is administered in conjunction with mechanical thrombus fragmentation and/or aspiration thrombectomy (mechanical thrombectomy) and/or rheolytic thrombectomy.

In one embodiment, the composition for use is formulated and administered as a solution, an injection, a pill, a tablet, a capsule, a suppository, an oral solution, a buccal, a granule, a powder, a suspension, a spray, an ointment, drops, or patches.

In one aspect, the present invention relates to a portable medicine injection device comprising a composition for use as defined herein.

In one embodiment, the medicine injection device is a pen-type drug delivery device; preferably a disposable pen-type drug delivery device.

In one embodiment, the portable medicine injection device can be used by an untrained person, such as the patient itself.

In one embodiment, the portable medicine injection device is used for the treatment and/or prevention of a thrombotic disorder, wherein the thrombotic disorder is as defined herein.

In one aspect, the present invention relates to a kit comprising:
a first container comprising the composition for use according to the present invention;
optionally, a second container comprising a composition comprising a pharmaceutically acceptable diluent; and
optionally, means for the intravenous administration, subcutaneous administration, intraperitoneal administration and/or intramuscular administration of said composition for use, such as syringes;
wherein the container is selected from a group comprising vials, bottles, pre-filled syringes, and ampoules.

In one embodiment, the composition for use is provided in a form selected from: an injection concentrate, a pre-diluted solution, and a lyophilized powder for reconstitution; preferably an injection concentrate.

In one embodiment, the kit is used for the treatment and/or prevention of a thrombotic disorder, wherein the thrombotic disorder is as defined herein.

In one aspect the present invention relates to a pharmaceutical composition comprising the composition for use as defined herein.

In one aspect the present invention relates to a pharmaceutical composition comprising the composition for use as defined herein for use in a method of treatment and/or prevention of a thrombotic disorder in a patient in need thereof.

According to this aspect, the thrombotic disorder, the composition, the administration and the patient are as defined herein.

In one aspect, the present invention relates to a method for the treatment and/or prevention of a thrombotic disorder, comprising the administration of a therapeutically effective amount of a composition comprising isothiocyanate, wherein the isothiocyanate is selected from 2-phenylethyl-isothiocyanate (PEITC), benzyl-isothiocyanate (BITC), and/or combinations of PEITC and BITC, wherein, optionally, the composition further comprises one or more further isothiocyanate(s), for use according to the present invention to a patient in need thereof.

According to this aspect, the thrombotic disorder, the composition, the administration and the patient are as defined herein.

In one aspect, the present invention relates to the use of a composition comprising isothiocyanate, for the manufacture of a medicament for the treatment and/or prevention of a thrombotic disorder, wherein the isothiocyanate is selected from 2-phenylethyl-isothiocyanate (PEITC), benzyl-isothiocyanate (BITC), and/or combinations of PEITC and BITC, wherein, optionally, the composition further comprises one or more further isothiocyanate(s).

According to this aspect, the thrombotic disorder, the composition for use, the administration and the patient are as defined herein.

In one aspect, the present invention relates to a composition for use as defined herein, for the prevention of coagulation in a blood sample.

### DETAILED DESCRIPTION

In one embodiment, the term "patient" relates to a human or an animal, preferably a human.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "thrombotic disorder", as used herein, refers to a medical condition characterized by the abnormal formation of blood clots (thrombi) within blood vessels, which can impede or completely block the flow of blood through the circulatory system. Thrombotic disorders can occur in both the arterial and venous systems, leading to a range of serious health complications depending on the location and size of the clot. Common thrombotic disorders include deep vein thrombosis, pulmonary embolism, myocardial infarction (heart attack), and ischemic stroke. The term "thrombotic disorders" may also be referred to "thrombotic diseases" and also includes thrombosis and diseases that are related to thrombosis. Thrombotic disorders are often associated with a combination of risk factors, including genetic predispositions (such as Factor V Leiden mutation), acquired conditions (such as antiphospholipid syndrome), lifestyle factors (such as smoking, obesity, and prolonged immobility), and underlying medical conditions (such as cancer, diabetes, and cardiovascular diseases).

In one embodiment, the patient is a human with a high predisposition for thrombotic disorders, such as a human exposed to risk factors, including genetic predispositions, acquired conditions (such as antiphospholipid syndrome), lifestyle factors (such as smoking, obesity, and prolonged immobility), and underlying medical conditions (such as cancer, diabetes, and cardiovascular diseases). Genetic predispositions include, but are not limited to, Factor V Leiden mutation, prothrombin G20210A mutation, protein C deficiency, protein S deficiency, hyperhomocysteinemia, plasminogen Activator Inhibitor-1 (PAI-1) polymorphisms, inherited dysfibrinogenemia, thrombomodulin mutations and other genetic variants in coagulation factors, such as Factor VIII, Factor IX, and Factor XI, that can also contribute to an increased risk of thrombotic disorders.

The term "thrombosis", as used herein, refers to the formation or presence of a blood clot within a blood vessel, which can impede or completely block the flow of blood through the circulatory system. This condition can occur in any part of the body and can lead to serious medical complications depending on the location of the clot. For example, thrombosis in the coronary arteries can result in a heart attack, while thrombosis in the cerebral arteries can lead to a stroke. Thrombosis can be classified into two main types: venous thrombosis, which occurs in veins, and arterial thrombosis, which occurs in arteries. The underlying causes of thrombosis can include endothelial injury, abnormal blood flow, and hypercoagulability, collectively known as Virchow's triad. Effective management of thrombosis often involves the use of anticoagulants, thrombolytic therapy, and mechanical interventions to restore normal blood flow and prevent further complications.

The term "venous thrombosis", as used herein, refers to the formation of a blood clot within a vein, which can cause swelling, pain, and redness in the affected area, and may lead to complications such as deep vein thrombosis or pulmonary embolism if the clot dislodges and travels to the lungs.

The term "arterial thrombosis", as used herein, refers to the formation of a blood clot within an artery, which can restrict or block the flow of oxygen-rich blood to vital organs and tissues, potentially resulting in conditions such as myocardial infarction (heart attack), ischemic stroke, or peripheral artery disease.

The term "thrombus", as used herein, refers to a blood clot that forms in a blood vessel and remains at the site of its formation, which can obstruct blood flow and cause tissue damage or necrosis if not properly managed.

The term "embolus", as used herein, refers to a blood clot that has been carried in the bloodstream to lodge in a vessel and cause an embolism, potentially leading to life-threatening conditions such as stroke, heart attack, or pulmonary embolism.

The term "activation of platelets", as used herein, refers to the process by which platelets become primed to participate in blood clotting, typically involving changes in shape, release of granule contents, and increased adhesiveness. Activation of platelets plays an important role in the formation of a stable blood clot to prevent excessive bleeding, however, is also involved in the formation of thrombi and emboli. Platelets (PLTs) are anucleated cells derived from megakaryocytes. For a long time, it was considered that the main function of PLTs was to reduce blood loss upon injury. However, it is now apparent that in addition to hemostasis, thrombus formation and atherosclerosis, PLTs also play a role in inflammation and the immune response. Under physiological conditions, PLT activation is prevented by prostaglandin I₂ (PGI₂) and NO, which are secreted from the vascular endothelium. Upon injury or in other pathological settings, PLTs can be activated by binding of collagen, fibrinogen, thrombin, the von Willebrand Factor (vWF), ADP, thromboxane A₂ (TXA₂), immunoglobulin G antibodies (IgGs) or immune complexes to receptors on the PLT surface. PLT activation leads to the initiation of signaling cascades that result in PLT shape change and degranulation, which promotes further PLT activation. Furthermore, PLT activation leads to expression of P-Selectin (CD62P), CD63 and CD40L at the PLT surface, the first two being markers of PLT degranulation. PLT activation also results in enhanced ROS levels, which further activate PLT, whereas too high ROS levels inhibit PLT activation. Ultimately, PLT activation results in PLT aggregation, which is needed for vascular wound closure, however, is detrimental, when no wound is present and a thrombus is built instead. Therefore, when PLT function is altered, risk for thrombosis is increased with potentially lethal outcomes in the form of stroke, myocardial infarction or pulmonary embolism.

The term "degranulation of platelets", as used herein, refers to the release of granule contents from platelets, which includes various substances that promote blood clotting and inflammation, such as ADP, serotonin, and thromboxane A2, playing a crucial role in the hemostatic response.

The term "aggregation of platelets", as used herein, refers to the clumping together of platelets in the blood, which is a crucial step in the formation of a blood clot, helping to seal wounds and prevent blood loss, but can also contribute to pathological clot formation in conditions such as thrombosis.

The term "capable of preventing", as used herein, refers to having the ability to stop or hinder a specific process or action, such as in "capable of preventing activation of platelets", indicating that a substance or intervention can effectively inhibit the initial steps of platelet activation and subsequent clot formation.

The term "anti-coagulant", as used herein, refers to a substance that prevents blood clotting by inhibiting various factors in the coagulation cascade, thereby reducing the risk of clot formation and helping to manage conditions such as thrombotic disorders.

The term "platelet-active drug", as used herein, refers to a medication that affects platelet function by inhibiting their activity, and is used to prevent and/or treat conditions related to abnormal platelet aggregation, such as heart attack, stroke, and other thrombotic disorders.

The term "administration", as used herein, refers to the application of a composition, a compound or a pharmaceutical composition thereof to a patient.

The term "subcutaneous administration", as used herein, refers to the administration of a substance into the tissue layer between the skin and the muscle.

The term "intravenous administration", as used herein, refers to the delivery of a substance directly into a vein, allowing for rapid absorption and immediate effect, commonly used for medications, fluids, and nutrients in clinical settings.

The term "intraperitoneal administration", as used herein, refers to the delivery of a substance into the peritoneal cavity, the area that contains the abdominal organs.

The term "intramuscular administration", as used herein, refers to the delivery of a substance directly into a muscle, allowing for slower absorption compared to, for example, intravenous administration.

The term "administration via a catheter", as used herein, refers to the use of a catheter to deliver medication directly to site of interest. Administration via a catheter is, for example, used during catheter lysis.

The term "catheter lysis", as used herein, refers to the use of a catheter to deliver medication directly to a blood clot to dissolve it, a minimally invasive procedure often used to treat acute thrombotic events such as deep vein thrombosis or pulmonary embolism.

In one embodiment, the composition for use is administered in conjunction with mechanical thrombus fragmentation and/or aspiration thrombectomy (mechanical thrombectomy) and/or rheolytic thrombectomy. In conjunction with is to be understood that the mechanical thrombus fragmentation and/or aspiration thrombectomy and/or rheolytic thrombectomy can either be performed simultaneously or subsequently with the administration of the composition for use. It is also possible that the time point(s) of the mechanical thrombus fragmentation and/or aspiration thrombectomy and/or rheolytic thrombectomy is independent from the administration of the composition for use. Moreover, it is not necessary that the mechanical thrombus fragmentation and/or aspiration thrombectomy and/or rheolytic thrombectomy is performed with each administration of the composition for use. Rather, the mechanical thrombus fragmentation and/or aspiration thrombectomy and/or rheolytic thrombectomy is performed one or more times, optionally only once, depending on the patient's situation and can be repeated if needed.

The term "aspiration thrombectomy", as used herein, refers to a minimally invasive medical procedure that removes a blood clot by suction through a catheter. This technique is commonly used in emergency settings to quickly restore blood flow in cases of acute ischemic stroke, myocardial infarction, or other thrombotic occlusions in both arterial and venous systems. During the procedure, a specialized catheter is navigated to the site of the thrombus under imaging guidance. Once in position, the catheter creates a vacuum or suction force that aspirates the clot, effectively removing it from the blood vessel. Aspiration thrombectomy is particularly advantageous because it can rapidly clear large clots, thereby minimizing tissue damage and improving clinical outcomes. The procedure can be performed alone or in combination with other interventional techniques, such as stent placement or balloon angioplasty, to ensure the vessel remains open and blood flow is adequately restored. Additionally, aspiration thrombectomy is associated with lower risks of distal embolization and vessel injury compared to mechanical thrombectomy methods, making it a valuable tool in the management of acute thrombotic events.

The term "rheolytic thrombectomy", as used herein, refers to a minimally invasive medical procedure that removes a blood clot using a high-speed saline jet to break up the clot and remove it from the blood vessel. This technique is particularly useful in treating acute thrombotic events where rapid restoration of blood flow is critical, such as in cases of peripheral arterial disease, deep vein thrombosis, or acute limb ischemia. During the procedure, a catheter equipped with a high-pressure saline jet is inserted into the affected blood vessel. The saline jet creates a vacuum effect that fragments the thrombus into smaller particles, which are then aspirated out of the vessel. Rheolytic thrombectomy offers several advantages, including reduced risk of distal embolization, minimal damage to the vessel wall, and the ability to quickly restore blood flow. It is often used in conjunction with other therapies, such as anticoagulants or thrombolytics, to enhance the overall effectiveness of the treatment and improve patient outcomes.

The term "mechanical thrombus fragmentation", as used herein, refers to the physical breaking up of a blood clot using a mechanical device, a technique employed to restore blood flow in occluded vessels and reduce the risk of complications associated with thrombi.

The term "portable medicine injection device", as used herein, refers to a device that can be easily carried and used to inject medication, providing convenience and mobility for patients who require regular or emergency medication administration.

The term "pen-type drug delivery device", as used herein, refers to a device shaped like a pen that is used to deliver medication, typically through injection, offering ease of use, precise dosing, and portability for patients managing a disease such as a thrombotic disorder.

The term "in an amount sufficient to prevent", such as in "in an amount sufficient to prevent platelet activation", as used herein, refers to a quantity of a substance that is adequate to stop a specific process or action from occurring, ensuring the desired therapeutic effect, such as preventing the activation of platelets and subsequent clot formation. The skilled person would know how to determine and chose an appropriate amount sufficient to prevent based on the specific thrombotic condition.

The term "benzyl-isothiocyanate (BITC)", as used herein, refers to a naturally occurring compound, characterized by the presence of a benzyl group attached to an isothiocyanate functional group. In the context of the present invention BITC preferably refers to a compound according to the following formula:

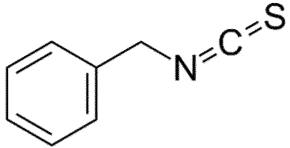

The term "phenylethyl-isothiocyanate (PEITC)", as used herein, refers to an organic compound that consists of a phenylethyl group bonded to an isothiocyanate group. In the context of the present invention PEITC preferably refers to a compound according to the following formula:

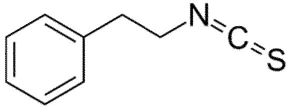

The term "allyl-isothiocyanate (AITC)", as used herein, refers to a chemical compound that contains an allyl group linked to an isothiocyanate group. In the context of the present invention AITC preferably refers to a compound according to the following formula:

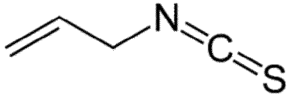

The term "treatment", as used herein, refers to the process of providing a subject with a pharmaceutical treatment, e.g., the administration of a drug, such that at least one symptom of the disease is decreased. Treatment of a disease can be improving the disease and/or curing the disease.

The term "prevention", as used herein, refers to the process of providing a subject with a pharmaceutical treatment, e.g., the administration of a drug, such that at least one symptom of the disease is prevented from occurring.

The term "pharmaceutically acceptable carrier, diluent and/or excipient" refers to a non-toxic, inert, solid, semi-solid, or liquid diluent material or formulation auxiliary of any type. "Pharmaceutically acceptable" in this context is meant to designate that said carrier is compatible with the other ingredients of the pharmaceutical composition and not harmful to the patient that the pharmaceutical composition is administered to. Examples of pharmaceutically acceptable carriers include, but are not limited to, water, water-propylene glycol solutions, or aqueous polyethylene glycol solutions.

The term "ITC mix", as used herein, preferably refers to a combination of PEITC, BITC and AITC. These components are preferably mixed in a mass ratio of PEITC : AITC : BITC is in the range from about 100 : 10 : 1 to about 1 : 10 : 100, more preferably in the range from about 50 : 5 : 1 to about 1 : 5 : 50, even more preferably in the range from about 10 : 5 : 1 to about 1 : 5 : 10, even more preferably the mass ratio is about 1 : 3.1 : 4.1, most preferably the mass ratio is about 1 : 3.13 : 4.13. In preferred embodiments, the ITC mix according to the present invention can be prepared by mixing 37.9% allyl isothiocyanate (AITC), 50% benzyl isothiocyanate (BITC) and 12.1% phenylethyl isothiocyanate (PEITC).

### BRIEF DESCRIPTION OF THE FIGURES

The present invention is now further described by reference to the following figures.

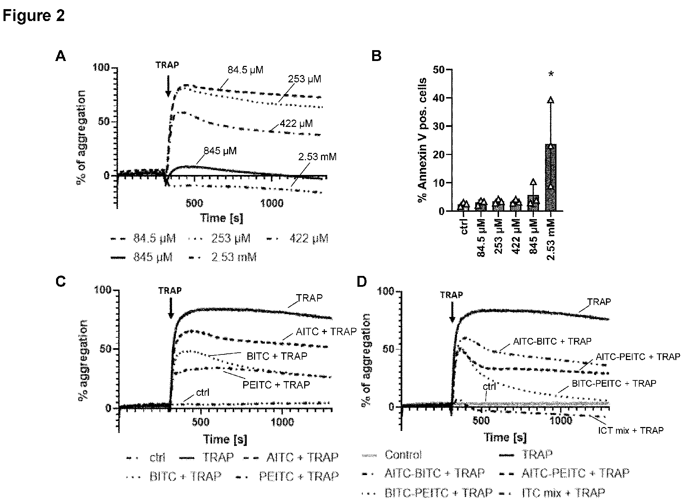

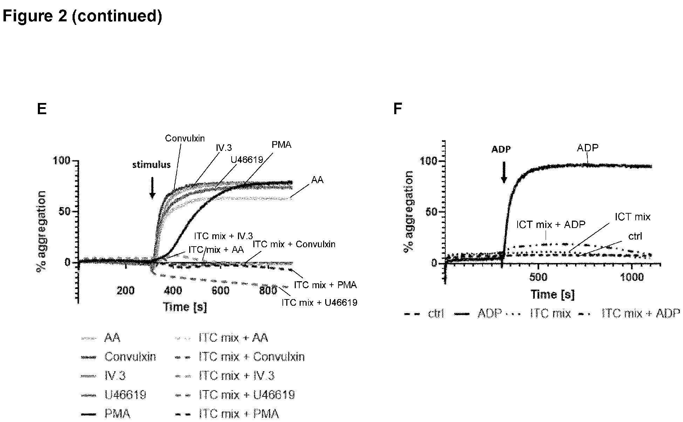

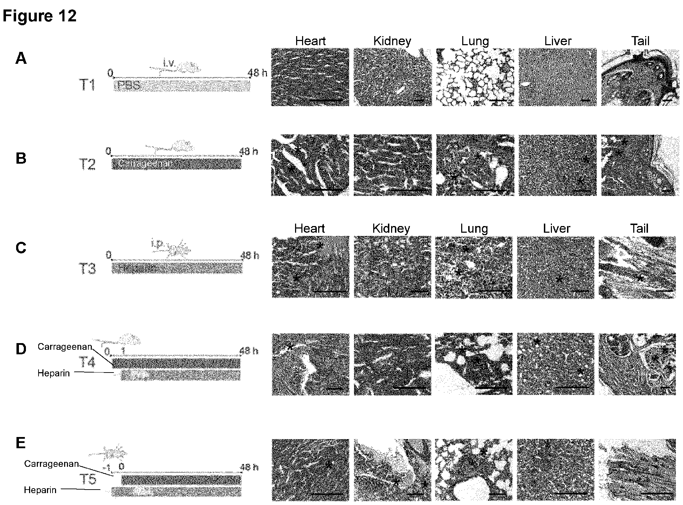

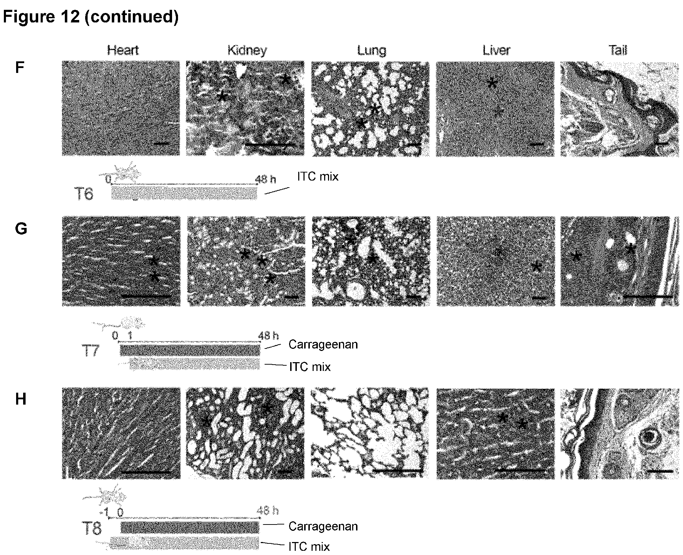

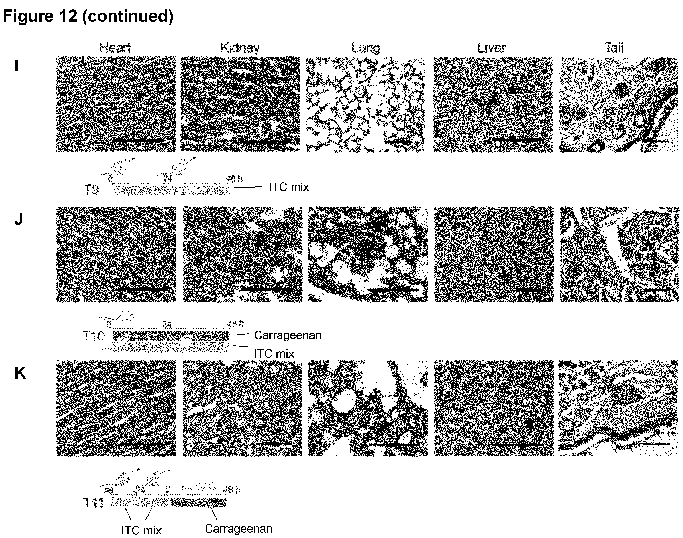

All methods mentioned in the figure descriptions below were carried out as described in detail in the examples.

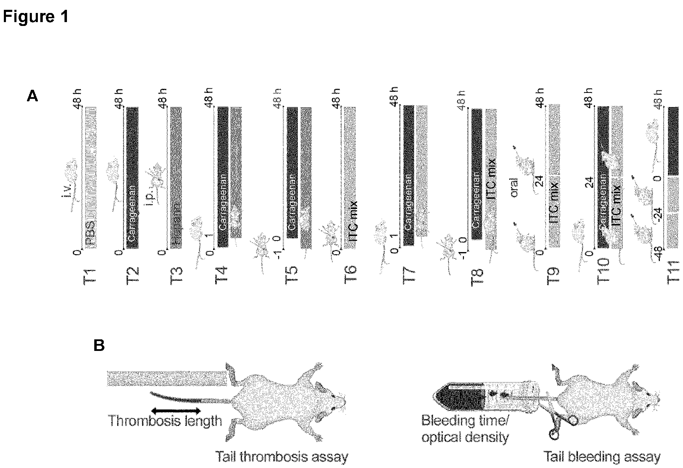

Figure **1****.** Mouse experiments setup.
   A: Murine treatment strategy. T1: PBS i.v., T2: Carrageenan i.v., T3: Heparin i.p., T4: Carrageenan i.v. + Heparin i.p. (1 h post Carrageenan), T5: Heparin i.p. + Carrageenan i.v. (1 h post Heparin), T6: ITC mix i.p., T7: Carrageenan i.v. + ITC mix i.p. (1 h post Carrageenan), T8: ITC mix i.p. + Carrageenan i.v. (1 h post ITC mix), T9: ITC mix p.o. (0 h and 24 h), T10: Carrageenan i.v. + ITC mix p.o. (0 h and 24 h), T11: ITC mix p.o. (48 h and 24 h prior to Carrageenan) + Carrageenan i.v.. **B:** Schematic drawing of tail thrombosis assay and tail bleeding assay
**Figure 2****.** ITCs prevent PLT aggregation irrespective of the aggregation-inducing stimulus. **A:** Aggregometry of PLTs in Tyrode buffer treated with different concentrations of the ITC mix at 0 s and induction of aggregation with TRAP at 300 s. N=3-5 obtained from different donors. **B:** Annexin V staining of PLTs treated with different concentrations of the ITC mix for 5 min in the aggregometer. N=3 obtained from different donors. One-Way ANOVA with Tukey's post-hoc test. * = p≤0.05, all samples were compared with each other, * is significantly different to all other samples. **C:** Aggregometry of PLTs in Tyrode buffer treated with single ITCs and induction of aggregation with TRAP at 300 s. N=3-5 from different donors. **D:** Aggregometry of PLTs treated with a combination of two ITCs at 0 s and induction of aggregation with TRAP at 300 s. N=3-6 from different donors. **E:** Representative aggregometry measurements of PLTs in Tyrode buffer treated with the ITC mix at 0 s and induction of aggregation with arachidonic acid (AA), convulxin (CVX), IV.3+Fab (IV.3), U46619, or PMA at 300 s. N=3-4 from different donors. For better visibility, representative graphs without error bars are shown in this graph. **F:** Aggregometry of PRP treated with the ITC mix at 0 s and induction of aggregation with ADP at 300 s. N=3 from different donors.

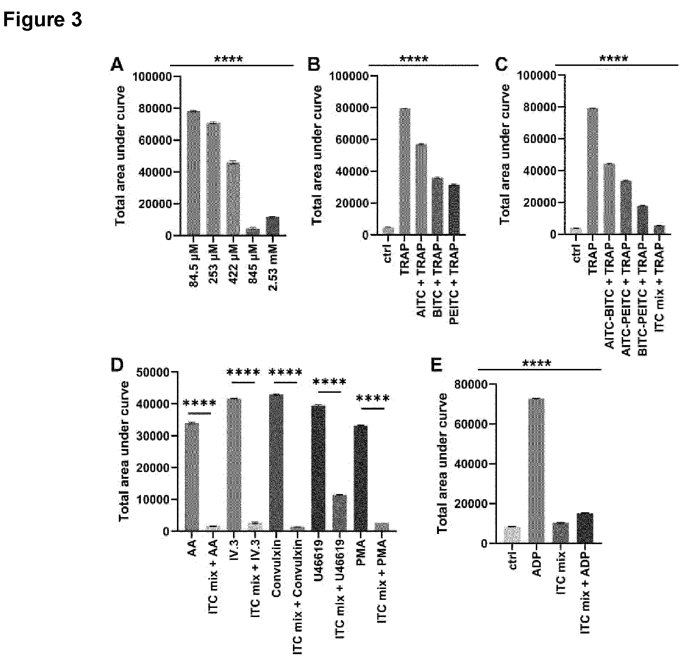

**Figure 3****.** Area under the curve analyses of aggregometry graphs depicted in Figure 2. **A:** Area under the curve aggregometry measurement of PLTs in Tyrode buffer treated with different concentrations of the ITC mix at 0 s and induction of aggregation with TRAP at 300 s. N=3-5 obtained from different donors. **** = p≤0.0001, all samples were compared with each other, **** is significantly different to all other samples. **B:** Area under the curve of the aggregometry measurement of PLTs in Tyrode buffer treated with single ITCs and induction of aggregation with TRAP at 300 s. N=3-5 from different donors. **** = p≤0.0001, all samples were compared with each other, **** is significantly different to all other samples. **C:** Area under the curve of the aggregometry measurement of PLTs treated with a combination of ITCs and induction of aggregation with TRAP at 300 s. N=3-6 from different donors. **** = p≤0.0001, all samples were compared with each other, **** is significantly different to all other samples. **D:** Area under the curve of the aggregometry measurement of PLTs in Tyrode buffer treated with the ITC mix and induction of aggregation with arachidonic acid (AA), IV.3+Fab (IV.3), convulxin (CVX), U46619, or PMA at 300 s. N=3-4 from different donors. **** = p≤0.0001. Unpaired Student's t-tests between agonist-treated samples and ITC mix-pretreated samples. **E:** Area under the curve of the aggregometry measurement of PRP treated with the ITC mix and induction of aggregation with ADP at 300 s. N=3 from different donors. **** = p≤0.0001, all samples were compared with each other, **** is significantly different to all other samples.

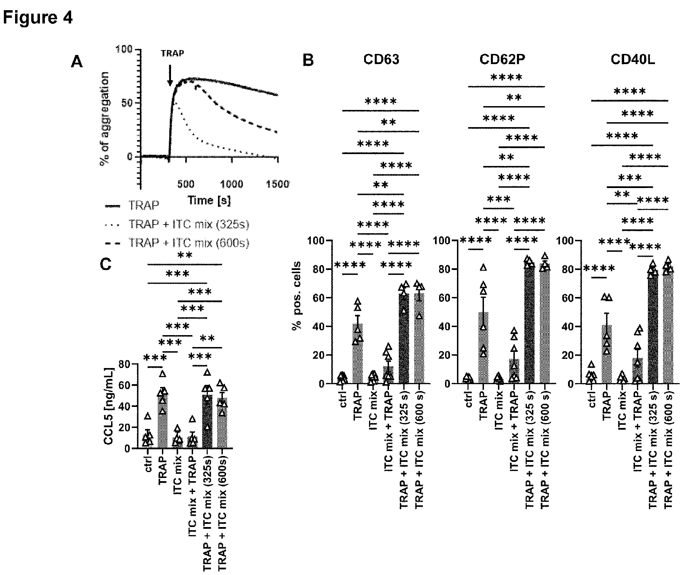

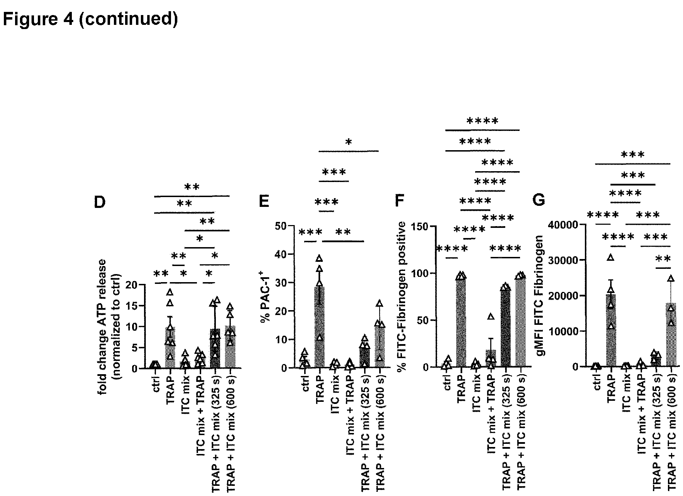

**Figure 4****.** ITCs reverse PLT aggregation but not PLT degranulation.
   **A:** PLT were treated with the ITC mix or TRAP at the indicated timepoints. N=5 from different donors. **B:** Flow cytometry measurements of PLT activation markers CD63, CD62P and CD40L. N=4-7 from different donors. One-Way ANOVA with Tukey's post-hoc test ** = p ≤ 0.005, *** = p ≤ 0.0005, **** = p ≤ 0.0001. **C:** CCL5 ELISA after respective treatments in the aggregometer. N=5 from different donors. One-Way ANOVA with Tukey Post-hoc test ** = p ≤ 0.005, *** = p ≤ 0.0005, **** = p ≤ 0.0001. **D:** ATP release of PLTs after respective treatments in the aggregometer, normalized to control. N=6 from different donors. One-Way ANOVA with Tukey's post-hoc test. * = p ≤ 0.05, ** = p ≤ 0.005. **E:** Flow cytometry measurements of activated GPIIb/IIIa (PAC-1) on PLTs after respective treatments. N=5 from different donors. One-Way ANOVA with Tukey's post-hoc test * = p ≤ 0.05, ** = p ≤ 0.005, *** = p ≤ 0.0005. **F-G:** Flow cytometry measurement of fibrinogen binding to PLTs upon treatment with ITCs. **F:** percentage of PLTs that bound FITC-labelled fibrinogen, **G:** gMFI of PLTs. N=4 from different donors. One-Way ANOVA with Tukey's post-hoc test ** = p ≤ 0.005, *** = p ≤ 0.0005, **** = p ≤ 0.0001.
**Figure 5****.** ITCs reverse PLT aggregation irrespective of the aggregation-inducing stimulus. PLT were activated using AA, U46619, CVX, IV.3 or PMA at 300 s. 25 s or 300 s thereafter, the ITC mix was added. N=3 from different donors.

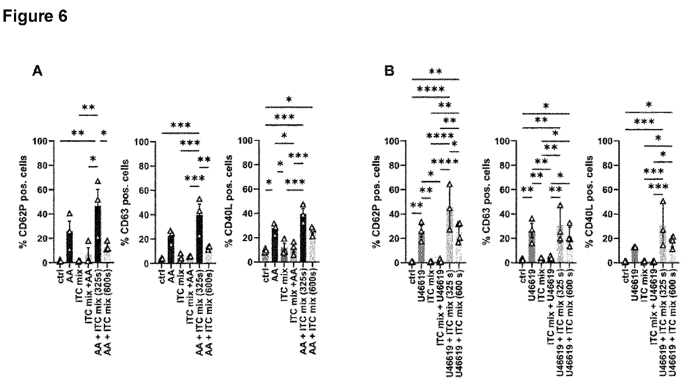

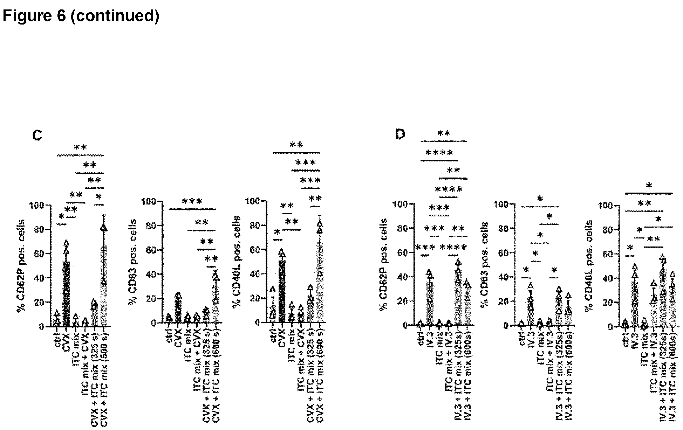

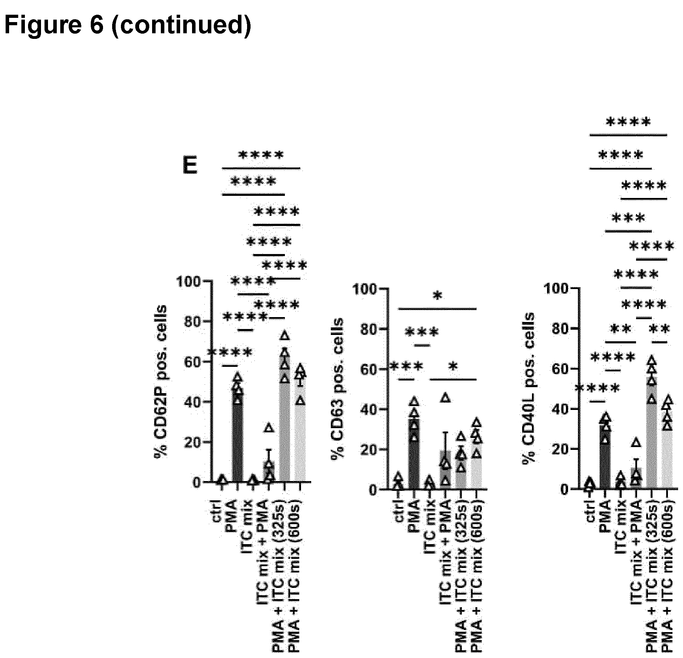

**Figure 6****.** Irrespective of the aggregation-inducing stimulus, ITCs prevent PLT activation. Flow cytometry measurements of PLT activation markers CD63, CD62P and CD40L upon **A:** PLT activation with AA and treatment with the ITC mix at indicated timepoints. **B:** PLT activation with U46619 and treatment with the ITC mix at indicated timepoints. N=4-7 from different donors. **C:** PLT activation with CVX and treatment with the ITC mix at indicated timepoints. N=4-7 from different donors. **D:** PLT activation with IV.3 and treatment with the ITC mix at indicated timepoints. N=4-7 from different donors. **E:** PLT activation with PMA and treatment with the ITC mix at indicated timepoints. N=4-7 from different donors. One-Way ANOVA with Tukey Post-hoc test * = p ≤ 0.05, ** = p ≤ 0.005, *** = p ≤ 0.0005, **** = p ≤ 0.0001.
**Figure 7****.** Dense granule release from PLTs measured via Serotonin release.
   Serotonin release of PLTs after respective treatments in the aggregometer. N=6 from different donors. One-Way ANOVA with Tukey Post-hoc test.
**Figure 8****.** ITCs inhibit PLT signaling cascades associated with PLT activation.
   **A:** Representative Western blots showing (phospho)AKT levels with actin as loading controls. **B:** Quantification of AKT protein levels, **C:** quantification of pAKT protein levels N=4-8 from different donors. One-Way ANOVA with Tukey's post-hoc test ** = p ≤ 0.005, *** = p ≤ 0.0005, **** = p ≤ 0.0001. **D-E:** ITC effect on the kinome of resting and activated PLTs. Coral kinome trees display the activity of protein kinases upon **D:** ITC treatment compared to resting PLTs or E: TRAP + ITC mix compared to activated PLTs. N=4 from different donors. **F:** Reactome analysis by gprofiler of regulated PLT-relevant kinases upon ITC mix treatment. Pathways directly associated with PLT activation are highlighted in dark grey.

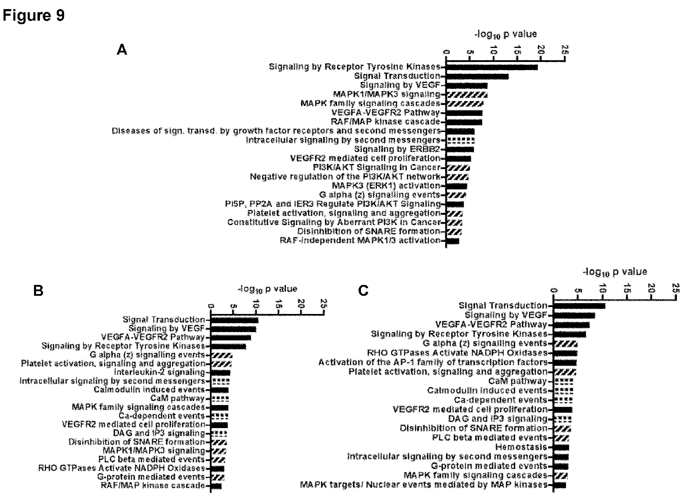

**Figure 9****.** Pathway analyses of significantly regulated kinases in PLTs upon treatment with ITCs.
   **A:** Top 20 hits in g:Profiler/Reactome for differentially regulated kinases between control and ITC mix treated PLTs. Diagonally striped bars indicate pathways associated with PLT activation, dotted bars indicate pathways directly associated with Ca²⁺-signaling. **B:** Top 20 hits in g:Profiler/Reactome for differentially regulated kinases between ITC mix + TRAP and TRAP- treated PLTs. Diagonally striped bars indicate pathways associated with PLT activation, dotted bars indicate pathways directly associated with Ca²⁺-signaling. **C:** Top 20 hits in g:Profiler/Reactome for differentially regulated kinases between TRAP + ITC mix (325 s) and TRAP- treated PLTs. Diagonally striped bars indicate pathways associated with PLT activation, dotted bars indicate pathways directly associated with Ca²⁺-signaling.

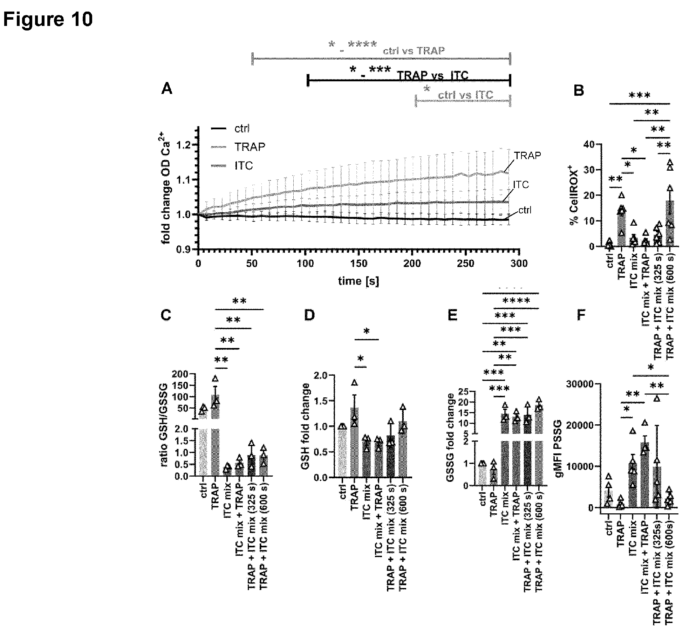

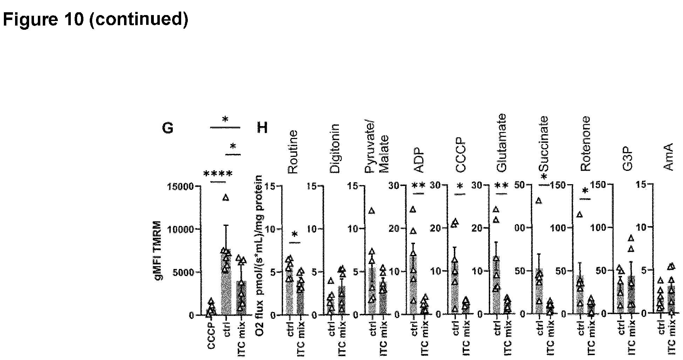

**Figure 10****.** ITCs affect PLT mitochondrial function.
   **A:** Fold change of intracellular Ca²⁺-levels of PLTs after respective treatments over time. PLTs were stained with Calbryte520^{™} AM and fluorescence was measured over the time of 5 minutes. N=5-6 from different donors. 2-Way ANOVA with Tukey's post-hoc test * = p ≤ 0.05, ** = p ≤ 0.005, *** = p ≤ 0.0005, **** = p ≤ 0.0001. Dark grey significance asterisks indicate comparison ctrl vs TRAP. Black significance asterisks indicate comparison TRAP vs ITC mix. Light grey significance asterisks indicate comparison ctrl vs ITC. **B:** CellROX+ PLT upon treatment with the ITC mix or TRAP. N=6 from different donors. One-Way ANOVA with Tukey's post-hoc test * = p ≤ 0.05, ** = p ≤ 0.005, *** = p ≤ 0.0005, **** = p ≤ 0.0001 **C-E:** (oxidized) glutathione levels in PLTs following respective treatments. **C:** GSH/GSSG ratio **D:** GSH fold change **E:** GSSG fold change. N=3 from different donors, One-Way-ANOVA with Tukey's post-hoc test * = p ≤ 0.05, ** = p ≤ 0.005, *** = p ≤ 0.0005, **** = p ≤ 0.0001. **F:** Assessment of protein S-glutathionylation (PSSG) following respective treatments. N=5 from different donors. Mixed-effects analysis with Tukey's post-hoc test * = p ≤ 0.05, ** = p ≤ 0.005. **G:** mitochondrial membrane potential following treatment with ITCs, CCCP served as positive control to disrupt mitochondrial membrane potential. N=7 from different donors, One-Way-ANOVA with Tukey's post-hoc test * = p ≤ 0.05, **** = p ≤ 0.0001. **H:** PLT respiration measured in a O2k respirometer following an adjusted SUIT-01 protocol left panel: routine respiration. Thereafter, respiration was measured following the addition of Digitonin, Pyruvate/Malate, ADP, CCCP, Glutamate, Succinate, Rotenone, G3P, and AmA. N=6 from different donors. Unpaired two-sided Student's t-test, * = p ≤ 0.05, ** = p ≤ 0.005.

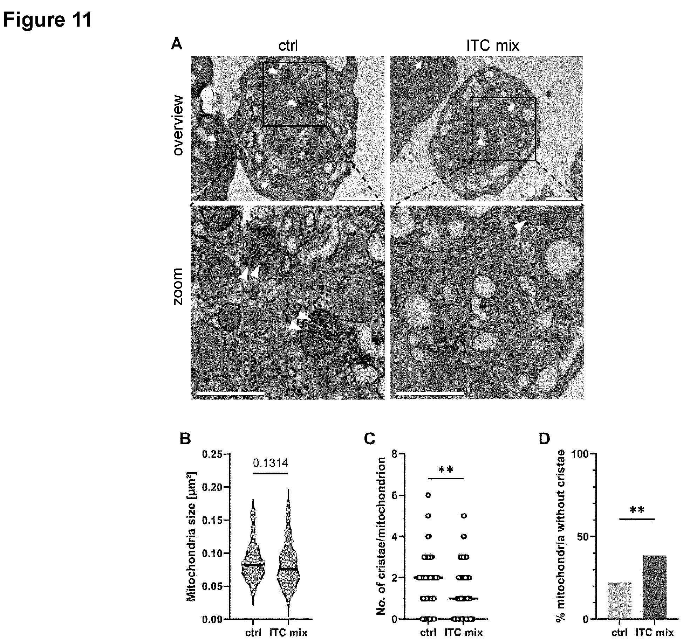

**Figure 11****.** PLT treatment with ITCs results in mitochondrial damage.
   **A:** Representative electron microscopy pictures of vehicle- and ITC mix-treated PLTs. Scale bar = 1000 µm in overview, and 500 nm in zoom. Arrows indicate mitochondria, arrowheads indicate cristae. N=4 from different donors. Images were acquired with a Jeol 1200 EXII electron microscope (Akishima) with a KeenViewII digital camera. **B:** analysis of mitochondrial size from electron microscopy pictures. Per condition, 130-150 mitochondria were analysed from 4 different donors. Black line indicates median. Welch's t-test ** = p ≤ 0.005. **C:** Per mitochondrion, number of visible cristae were counted. Two-tailed Mann-Whitney test ** = p ≤ 0.005
**Figure 12**. Immunohistological H&E staining of murine hearts, kidneys, lungs, livers, and tails upon indicated treatments.
   **A:** T1: PBS i.v., **B:** T2: Carrageenan i.v., **C:** T3: Heparin i.v., **D:** T4: Carrageenan i.v. + Heparin i.p. (1 h post Carrageenan), **E:** T5: Heparin i.p. + Carrageenan i.v. (1 h post Heparin), **F:** T6: ITC mix i.p., **G:** T7: Carrageenan i.v. + ITC mix i.p. (1 h post Carrageenan), **H:** T8: ITC mix i.p. + Carrageenan i.v. (1 h post ITC mix), **I:** T9: ITC mix p.o. (0 h and 24 h), **J:** T10: Carrageenan i.v. + ITC mix p.o. (0 h and 24 h), **K:** T11: ITC mix p.o. (48 h and 24 h prior to Carrageenan) + Carrageenan i.v.

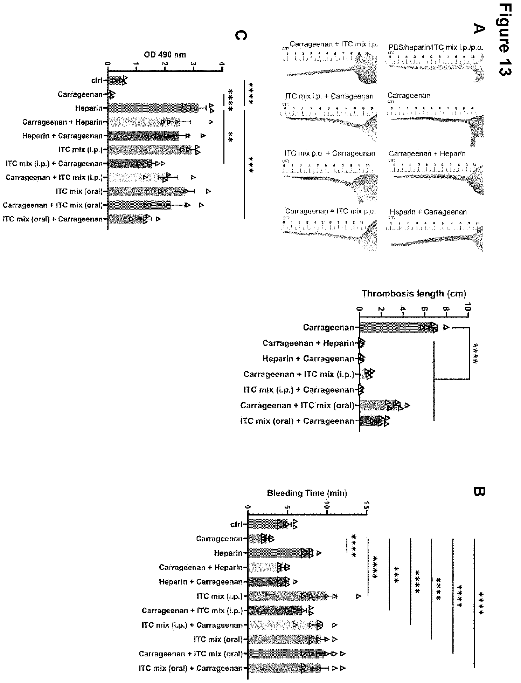

**Figure 13****.** Carrageenan-induced tail vein thrombosis is prevented by treatment with the ITC mix.
   **A:** left panel: representative pictures of carrageenan-induced murine tail vein thrombosis upon treatment with heparin or the ITC mix. Right panel: Length of carrageenan-induced tail vein thrombosis. N=5 mice per group, all samples were compared to the carrageenan treatment. One-Way ANOVA with Dunnett's post-hoc test, **** = p ≤ 0.0001. **B:** Bleeding time in tail bleeding assay upon induction of thrombosis with carrageenan and treatment with heparin or the ITC mix. N=5 mice per group, all samples were compared to the carrageenan treatment. One-Way ANOVA with Dunnett's post-hoc test, *** = p ≤ 0.0005, **** = p ≤ 0.0001. C: OD of bleeding assay after induction of thrombosis with carrageenan and treatment with heparin or the ITC mix. N=5 mice per group, all samples were compared to the carrageenan treatment. One-Way ANOVA with Dunnett's post-hoc test, ** = p ≤ 0.005, *** = p ≤ 0.0005, **** = ρ ≤ 0.0001.

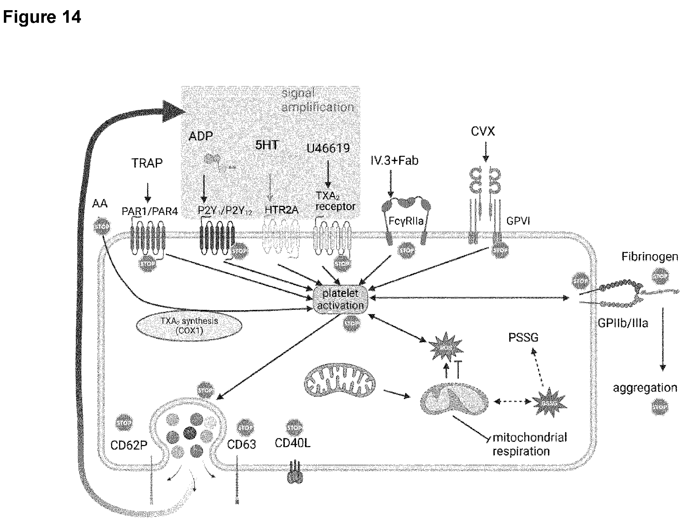

**Figure 14****.** Summary of ITC effects on PLTs
   ITCs prevented PLT activation and aggregation irrespective of the aggregation-inducing stimulus. In addition, ITCs also reversed PLT aggregation. These effects were reflected in the prevention and partial reversion of GPIIb/IIIa activation and fibrinogen binding of PLTs. On a molecular level, ITCs altered the redox-states of PLTs, resulting in elevated GSSG levels. Elevated GSSG levels were partially reflected in protein S-glutathionylation. In addition, ITCs altered mitochondrial structure, thereby inhibiting mitochondrial respiration.

### EXAMPLES

### Example 1

### Materials and Methods

### Platelet isolation

Informed written consent was obtained from all donors. Blood was taken from voluntary healthy donors in citrate tubes and centrifuged for 20 min at 150 g without brake. Platelets platelet rich plasma (PRP) was collected and 400 nM prostaglandin I₂ (PGI₂) was added to prevent platelet (PLT) aggregation due to isolation. After dilution with Tyrode buffer (137 mM NaCl, 2 mM KCl, 12 mM NaHCO₃, 300 µM NaH₂PO₄, 1 mM MgCl₂, 0.5 % BSA, pH 7.3), PRP was centrifuged for 10 min at 800 g without brake, the supernatant was removed and PLTs were resuspended in Tyrode buffer. PLT purity check was performed via flow cytometry.

### Flow cytometry

Flow cytometric analyses were performed using a flow cytometer. To check PLT purity, unfixed PLTs were stained for CD41-AF488 and CD45-APC.

For Annexin staining, an Annexin V staining kit was used according to manufacturer's instructions with small adjustments: only 1 µl Annexin V was used to stain PLTs, PI was not added, since PLTs are anucleate cells.

For all other flow cytometry experiments, PLTs were fixed in 1% paraformaldehyde (PFA) for 10 min. After removing PFA, PLTs were washed with 1% FCS + 2 mM EDTA in PBS (FACS buffer) and stained for 15 min in the dark using following antibodies in 1:100 dilution: CD62P-BV510, CD63-BV421, CD154-PE/Cy7, activated CD41/CD61. To measure fibrinogen binding, 50 µg/mL Fibrinogen-AF488 were added at the beginning of the experiment.

### Mediators

845 mM ITC stock solutions were prepared by mixing 37.9% allyl isothiocyanate (AITC), 50% benzyl isothiocyanate (BITC) and 12.1% phenylethyl isothiocyanate (PEITC) and diluting 1 : 10 in DMSO. TRAP-6 (25 µM), convulxin (CVX, 125 ng/mL), IV.3 (383 ng/mL, added at timepoint 0 s) in combination with Fab (15 µg/mL, added when aggregation was to be induced), arachidonic acid (AA, 100 µM) and ADP (100 µM) or phorbol 12-myristate 13-acetate (PMA, 1 µM) were used to induce PLT aggregation. All experiments were performed with PLTs in Tyrode buffer, apart from ADP-induced aggregation experiments, which were performed in PRP, since in Tyrode buffer no aggregation is induced by ADP.

For in vivo experiments, 37.9% AITC, 50% BITC and 12.1% PEITC were mixed, which will be also referred to as "ITC mix" in the following. For treatments, the ITC mix was diluted 1 : 10 in DMSO, thereafter diluted 1 : 10 in PBS and mice were treated intraperitoneally (i.p.) or orally (p.o.) as indicated.

### Aggregometry

For aggregometry measurements, 4×10⁷ PLTs in Tyrode buffer were used in a total volume of 200 µl in an APACT 4S PLUS aggregometer. In case of ADP-induced aggregation, 200 µl PRP was used. The APACT 4S PLUS aggregometer uses light transmission aggregometry (Born method) to determine PLT aggregation: light transmission through the sample correlates to the percentage of PLT aggregation. Before each experiment, baselines were set with Tyrode buffer for 100% aggregation. When performing aggregometry measurements with ADP-induced aggregation, platelet poor plasma (obtained by centrifugation of PRP for 5 min, 10000 g) was used to set the 100% aggregation baseline. PLTs in Tyrode buffer or PRP was set for the 0% aggregation baseline. Temperature was set to 37 °C, and stirring was set to 1000 rpm. Substances were added during the run as indicated.

### Western blot

1.2×10⁸ PLTs were incubated in 600 µl Tyrode buffer with mediators in the aggregometer for 900 s in total. After centrifugation at 430 g for 5 min, the pellet was resuspended in 100 µl lysis buffer with a Protease and Phosphatase Inhibitor Cocktail and PMSF and lysed on ice for 20 min. Sample concentration was measured using the Bradford assay according to manufacturer's instructions. Samples were boiled in SDS loading buffer for 5 min at 95°C before loading on 10% SDS gels and run at 90 V during stacking phase and 130 V during running phase. Blotting was performed for 90 min at 225 mA using the semi-dry blotting method on a PVDF membrane. Blocking and antibody incubation was performed in 5% milk in TBST or 5% BSA in TBST. Following primary antibodies were used in 1:1000 dilutions at 4°C overnight: anti-Akt, anti-phospho-Akt (S473) XP, as well as anti-β-actin (1:5000 dilution) as loading control. After washing, anti-mouse HRP or anti-rabbit HRP secondary antibodies were added in 1:5000 dilutions at room temperature for 1 h. Detection was performed with an ECL solution and the Chemidoc XRS+ System and analyzed using the ImageLab software.

### PamGene Kinase Assay

Kinase activities were assessed using the PamGene kinase assay. Therefore, after respective treatments in the aggregometer, PLTs were pelleted, snap-frozen in liquid nitrogen and sent to PamGene ('s Hertogenbosch, The Netherlands) for analysis of serine/threonine kinase (STK) and protein tyrosine kinase (PTK) activities. The specificity score (specificity for the kinase indicated) and kinase statistic (regulation of the kinase) were used to evaluate results. A final score above 1.2 was considered as statistically significant.

### ELISA

The ELISA MAX^{™} Deluxe Set Human CCL5 kit and the Serotonin ELISA kit were used to perform ELISA experiments according to manufacturer's protocols. 4×10⁷ PLTs in 200 µl Tyrode buffer were treated with the respective mediators in the aggregometer. After 900 s, samples were collected and centrifuged 5 min at 430 g with PGI₂ to avoid PLT activation during centrifugation. The supernatant was centrifuged at 10000 g, transferred to a new tube and stored at -80 °C until analysis. For the CCL5 ELISA, samples were diluted 1:200, whereas for the Serotonin ELISA samples were not diluted. Absorbance was measured using a Multiskan FC reader at 450 nm and 570 nm. Concentrations were calculated via four parameter logistic regression.

### ATP Assay

To measure ATP release from PLTs, supernatants were collected and measured using the CellTiter-Glo^{®} Luminescent Cell Viability Assay according to manufacturer's instructions, an assay that assesses the ATP content of a sample. Supernatants were used in a 1 : 10 dilution. Samples were analyzed in a Victor Nivo F.

### CellROX Assay

To assess intracellular ROS levels, PLTs were treated in the aggregometer, pelleted, resuspended in FACS buffer and stained with 5 µM CellROX^{™} Green reagent for 30 min at 37 °C. Afterwards, PLTs were washed and measured by flow cytometry.

### Measurement of mitochondrial respiration

Mitochondrial respiration of 3×10⁸ PLTs was measured in a high-resolution respirometer Oxygraph-2k using a substrate uncoupler inhibitor titration protocol. This protocol allows the analysis of the activity of the mitochondrial respiration complexes, by disrupting the cell membrane with digitonin and the subsequent addition of substances to specifically induce or inhibit mitochondrial respiration complexes. In brief, ITC mix-treated and vehicle-treated PLTs were washed, and resuspended in 2 mL MiR05 buffer (0.5 mM EGTA, 3 mM MgCl₂, 60 mM lactobionic acid, 20 mM taurine, 10 mM KH₂PO₄, 20 mM HEPES, 110 mM D-sucrose, 1 g/L BSA, pH=7.1). Firstly, routine respiration of intact PLTs was measured. Then, digitonin (10 µg per 100 µg protein), malate (5 mM) and pyruvate (1.6 mM), ADP (2.5 mM), carbonyl cyanide 4-(trifluoromethoxy)phenylhydrazone (CCCP, 500 nM), glutamate (10 mM), succinate (10 mM), rotenone (1 µM), glycerol 3-phosphate (10 mM) and antimycin A (2.5 µM) were added, once respiration was stabilized after the addition of the preceding substance. Concentrations for all substrates and inhibitors were adapted for measurements of platelet respiration. Data analysis was performed using the DatLab software and the recorded values of O₂ flux per volume were normalized to protein concentration inside the O2k-chambers.

### Mitochondrial membrane potential

To investigate mitochondrial membrane potential, after incubation with mediators, PLT were incubated with 400 nM tetramethylrhodamin-ethylester (TMRM) for 20 min at 37 °C. PLT were then washed and analyzed by flow cytometry. CCCP was used as positive control for disruption of mitochondrial membrane potential.

### GSH/GSSG measurements

Reduced glutathione (GSH) and oxidized glutathione (GSSG) levels were assessed using Promega's GSH/GSSG-Glo^{™} Assay according to manufacturer's instructions. Measurements of luminescence were performed in a Victor Nivo F. GSH/GSSG ratios were calculated as described in the manufacturer's protocol.

### Measurement of protein glutathionylation

Protein glutathionylation was measured using a S-glutathionylated protein detection kit, according to manufacturer's instructions with samples treated in the aggregometer. Sample detection was performed by flow cytometry.

### Measurement of Ca²⁺-levels in PLTs

PLT Ca²⁺-levels upon stimulation with TRAP or the ITC mix were assessed by staining PLTs with Calbryte-520AM for 1 h at 37 °C. PLTs were transferred into black 96-well plates with transparent well-bottoms. Mediators were added immediately before measurement in a Victor Nivo F. Fluorescence was measured every 7.5 s for 5 min.

### Electron Microscopy

For electron microscopy, 3×10⁸ PLTs were treated with the ITC mix, pelleted with PGI₂, fixed in 100 µL 2.5% glutaraldehyde in 0.1 M sodium cacodylate buffer and post-fixed in osmium tetroxide. Samples were then dehydrated with acetone and embedded in epoxy resin (Epon-812 according to Dalton).

After cutting ultrathin sections (50-60 nm), samples were stained in a Leica EM AC20 automatic contrasting instrument with UranyLess and 3% lead citrate.

Finally, images were acquired using a Jeol 1200 EXII electron microscope equipped with a KeenViewII digital camera at 60 kV and processed with the iTEM software package. Size of mitochondria was assessed using the QuPath v0.5.1 software.

### Animal handling

Healthy male BALB/C mice were purchased from the Pasteur Institute (Tehran, Iran) at the age of 7-9 weeks with a weight range of 25±5 g. The animals were housed in ventilated rooms with a 12/12 h light/dark cycle, at 22 °C and a relative humidity of 55%. Mice were given ad libitum access to standard compressed chow and water. Prior to the start of the study, the animals were kept for at least 2 weeks to acclimatize. Animal handling was performed in accordance with a revised Animals Directive 2010/63/EU of the European Union. In accordance with the Guidelines for the Care and Use of Animals, the Animal Ethics Committee of the Shahid Chamran University of Ahvaz (Iran) approved the procedures for handling the animals, as indicated by the permitted number EE/1401.2.24.218588/scu.ac.ir.

### Murine thrombosis model

Previous studies have revealed a range of doses for carrageenan injection that varied from 1 to 100 mg/kg to induce tail vein thrombosis. Experiments were performed after injections of 200 µg carrageenan diluted in physiological saline (10 mg/mL) into murine tail veins, resulting in a rapid tail color change, which became constant approximately after 48 h.

### In vivo ITC treatments

Intraperitoneal (i.p.) administration of the DMSO-diluted ITC mix diluted 1:2 in PBS caused strong toxicity in the animals, accompanied by tonic spasms, whereas 1:4, 1:6 and 1:8 dilutions resulted in weakness and depression. A 1 : 10 dilution did not show macroscopic pathological symptoms and was therefore chosen for further experiments. In the study, the i.p.-treated group received a volume of 100 µL, whereas the oral intake group was administered twice with double dose of 200 µL at 24 h intervals. For comparison the present inventors used a known anticoagulant, and selected the maximum well-tolerated dose of heparin in mice (2500 U/kg) to maximize its antithrombotic effect.

For the experiments, mice were randomly allocated to 11 experimental groups with 5 mice per group which are shown in **Table 1** and **Figure 1A****.** All experiments were conducted 48 hours after carrageenan treatment.

**Table 1 - Treatment strategy mice**

| | Treatment |
|---|---|
| T1 | PBS i.v. |
| T2 | Carrageenan i.v. |
| T3 | Heparin i.p. |
| T4 | Carrageenan i.v. + Heparin i.p. (1 h post Carrageenan) |
| T5 | Heparin i.p. + Carrageenan i.v. (1 h post Heparin) |
| T6 | ITC mix i.p. |
| T7 | Carrageenan i.v. + ITC mix i.p. (1 h post Carrageenan) |
| T8 | ITC mix i.p. + Carrageenan i.v. (1 h post ITC mix) |
| T9 | ITC mix p.o. (0 h and 24 h) |
| T10 | Carrageenan i.v. + ITC mix p.o. (0 h and 24 h) |
| T11 | ITC mix p.o. (48 h and 24 h prior to Carrageenan) + Carrageenan i.v. |

### Tail thrombosis assay

48 h after the respective treatments, mice were anesthetized by i.p. administration of a mixture of 25:5 mg/mL ketamine-xylazine (1 mL/kg body weight). Then, tail thrombosis length and appearance were measured and the tail bleeding assay was performed **(****Figure 1B**).

### Tail bleeding assay

Following anesthesia, 1 cm of the tail tip was amputated and the tail was immediately placed in pre-warmed, distilled water in a 50 mL Falcon tube **(****Figure 1B**). The bleeding time was recorded and an optical density measurement was taken 20 minutes after tail amputation at a wavelength of 490 nm. After the tail bleeding assay, mice were euthanized and tissue samples were collected for histopathological analysis.

### Histopathology evaluation

Hearts, livers, lungs, kidneys, and tails, were collected and fixed in a 10% formalin buffered solution for a minimum of 10 days. The tissue samples were then embedded in paraffin, sectioned into 5 µm, and stained with hematoxylin and eosin. The stained samples were observed under a microscope equipped with a camera to identify changes in the tissues. Images were captured under 10-40X magnification of five consecutive microscopic fields.

### Statistical analyses and figure preparation

All statistical analyses were performed using GraphPad Prism 10.2.3. After outlier analysis, the statistical significance was calculated. The statistical test is indicated in the figure legends of the corresponding figure. Significance was assumed for p ≤ 0.05.

### Example 2

All methods mentioned in this example were carried out as described in Example 1.

### ITC-treated platelets show less aggregation after stimulation

The aim of this example was to analyze whether a combination of ITCs can prevent PLT aggregation. Therefore, freshly isolated human PLTs were treated with different concentrations of the ITC mix. After 300 s TRAP was added to induce PLT aggregation. 845 µM ITC mix prevented PLT aggregation without inducing apoptosis, whereas lower concentrations of the ITC mix only partially prevented aggregation, and a higher concentration of the ITC mix was cytotoxic **(****Figure 2A** and **B** and **Figure 3A**). Then, the efficacy of the single ITCs to prevent PLT aggregation was assessed. For this, single ITCs (or a combination of two ITCs) were used in the same concentration as in the previous experiment. The results indicate that the single components are capable of preventing TRAP-induced PLT aggregation, even though the single components are not as efficient as the ITC mix in preventing TRAP-induced PLT aggregation **(****Figure 2C** and **D**, and **Figure 3B** and **C**). Next, it was investigated, whether the ITC mix can also prevent PLT aggregation induced by different stimulants than TRAP. Therefore, experiments were performed using arachidonic acid (AA, activation of TXA₂ synthesis), U46619 (TXA2 receptor agonist), IV.3+Fab (activation of the IgG receptor), convulxin (CVX, collagen receptor agonist), ADP (P2Y₂ and P2Y₁₂ agonist) or PMA (PCK activation) instead of TRAP to induce PLT aggregation. Indeed, the ITC mix prevented PLT aggregation upon aggregation induced by any stimulus tested **(****Figure 2E** and **F****,** and **Figure 3D** and **E****).** Together, these data demonstrate that ITCs can prevent PLT aggregation, irrespective of the aggregation-inducing stimulus.

### Example 3

All methods mentioned in this example were carried out as described in Example 1.

### ITCs reverse TRAP-induced PLT aggregation, but not degranulation

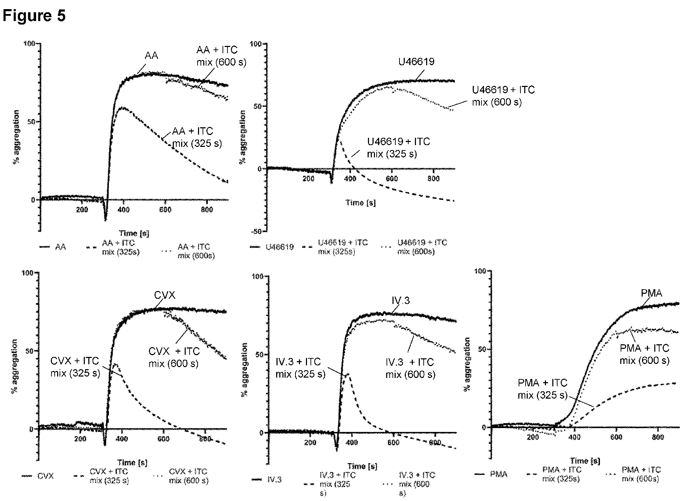

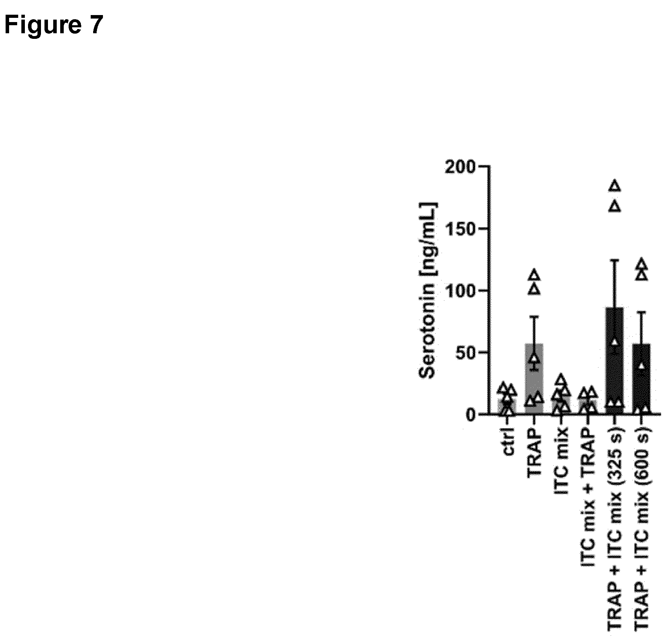

It was then investigated, whether ITCs can reverse PLT aggregation . To this end, after stimulation with TRAP (or other PLT agonists), ITCs were added 25 s or 300 s to the PLTs in the aggregometer. Addition of the ITCs at both timepoints was effective in reversing PLT aggregation **(****Figure 4A** and **Figure 5**). Having investigated PLT aggregation, PLT activation was analyzed, which precedes aggregation. ITCs efficiently prevented the surface expression of PLT activation and degranulation markers CD63, CD62P and CD40L, but did not reverse it **(****Figure 4B** and **Figure 6****).** Since all PLT agonists showed similar effects, the present inventors focused on TRAP-activated PLTs for the following experiments. To confirm that CD62P and CD63 surface expression is paralleled by PLT degranulation, CCL5 (a-granules), ATP and serotonin release levels (dense granules) were assessed. The results were in line with the flow cytometry data on PLT activation markers: While the ITC mix prevented CCL5 release when added prior to TRAP, it did not affect CCL5 release from activated PLTs **(****Figure 4C**). A similar effect was observed for dense granule release **(****Figure 4D** and **Figure 7**). In addition, GPIIb/IIIa activation as well as fibrinogen binding (**Figure 4E-G**) were investigated. The data demonstrate that ITCs could prevent and partially reverse integrin activation as well as fibrinogen binding to PLTs. Taken together, these data show that ITCs can reverse PLT aggregation and partially activation.

### Example 4

All methods mentioned in this example were carried out as described in Example 1.

### Activation of signaling pathways associated with PLT activation is prevented and reversed by ITC treatment

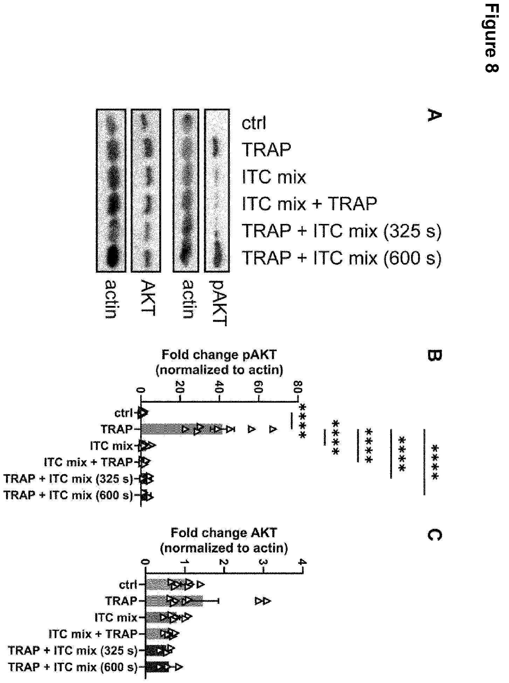

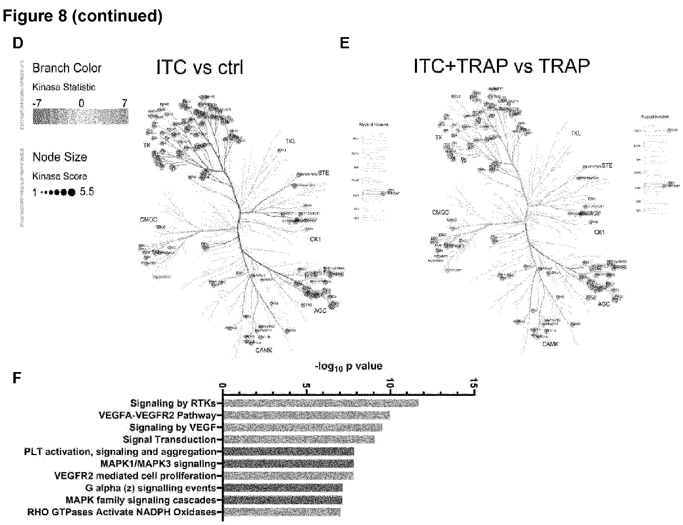

To investigate the effect of ITCs on PLTs on a molecular level, signaling pathways associated with PLT activation were analyzed. The ITC mix prevented and reversed TRAP-mediated phosphorylation of AKT **(****Figure 8A-C****).** In a broader approach, using the PamGene STK and PTK kinome assay it was found that the ITC mix effectively prevented and reversed the activity of a wide range of kinases associated with PLT activation and degranulation **(****Figure 8D** and **E** and **Figure 9**)**.** To obtain more specific results, the hits were filtered for kinases known to be expressed in PLTs using the PlateletWeb database and found several pathways directly linked to PLT activation to be regulated **(****Figure 8F****).** These data further indicate that ITCs can prevent and reduce signaling associated with PLT aggregation.

### Example 5

All methods mentioned in this example were carried out as described in Example 1.

### ITCs lead to oxidative stress and dysfunctional mitochondria in PLTs

Since the PamGene assay indicated that the Ca²⁺-signaling pathway was altered in ITC-treated PLTs **(****Figure 9**), and Ca²⁺-signaling is central in PLT activation, intracellular Ca²⁺-levels were measured. Cytoplasmic Ca²⁺-levels were enhanced in ITC-treated PLTs as compared to the control **(****Figure 10A**). However, they were significantly lower than in TRAP-activated PLTs. In addition to Ca²⁺, ROS is also an indicator and inducer of PLT activation. Therefore, ROS levels in ITC-treated PLTs were assessed. Indeed, ITCs reduced and prevented, when added 25 s post TRAP, TRAP-induced ROS production in PLTs **(****Figure 10B**). However, ROS levels were slightly increased in ITC-treated PLTs as compared to resting PLTs (only significant in t-test). Since enhanced ROS levels are a sign of oxidative stress, GSH/GSSG ratios were also investigated using a GSH/GSSG assay. GSH/GSSG ratios were decreased in ITC-treated samples, mainly caused by increased GSSG levels **(****Figure 10C-E**). Upon pretreatment of PLTs with ITCs, enhanced GSSG levels were paralleled by slightly enhanced protein S-glutathionylation (PSSG) levels **(****Figure 10F**).

Together, these data hint towards mitochondrial dysfunction. Therefore, the mitochondrial membrane potential was measured, which turned out to be decreased in ITC-treated PLTs as compared to control PLTs **(****Figure 10G**). In line with that, routine mitochondrial respiration was compromised in PLTs treated with ITCs, possibly caused by a reduced activity of the respiratory complexes I and II **(****Figure 10H**).

These data indicate that ITCs impair mitochondrial function of PLTs. Therefore, we assessed mitochondrial structure using transmission electron microscopy **(****Figure 11A**). Mitochondrial size was unchanged upon treatment with ITCs **(****Figure 11B**). However, ITCs decreased the number of cristae per mitochondrion **(****Figure 11C**), resulting in increased mitochondria without visible cristae **(****Figure 11D**).

In summary, these data indicate that ITCs induce mitochondrial dysfunction.

### Example 6

All methods mentioned in this example were carried out as described in Example 1.

### ITCs are effective in preventing carrageenan-induced tissue damage

Next, the efficacy of the ITC mix to prevent thrombosis in *vivo* was assessed. To that end, a murine carrageenan-induced tail vein thrombosis model was used, in which injections of 200 µg carrageenan resulted in tail necrosis, liver cell swelling, plaques of pulmonary thrombosis, and myocardial necrosis **(****Figure 12A-B**). Heparin (2500 U/kg) was used as a positive control for anticoagulation, and caused hemorrhage and hyperemia in the heart, lung, tail, and liver, along with hepatocyte swelling **(****Figure 12C**). Administration of heparin 1 h before or after carrageenan-induced tail vein thrombosis effectively prevented carrageenan-induced heart and tail muscle necrosis. However, some mice still exhibited bleeding and organ congestion, along with pulmonary thromboembolism **(****Figure 12D-E**). The identified lesions, such as cell swelling, may be ascribed to the transient effects of heparin injection on the evaluated organs. Intraperitoneal (i.p.) injections of the ITCs decreased carrageenan-induced tail necrosis and prevented cardiac necrosis and pulmonary thromboembolism, as well as peripheral thrombotic effects **(****Figure 12F-H**). In addition, tail necrosis incidence was significantly reduced, and thrombosis development in the heart was prevented by the oral administration of the ITCs **(****Figure 12I-K**). However, signs of lung congestion and swelling in liver and kidney cells were observed in i.p.-treated mice, whereas upon oral treatment, some carrageenan-induced pathological complications were still observed in the lungs and kidneys. The immunohistochemical findings are summarized in **Table 2.**

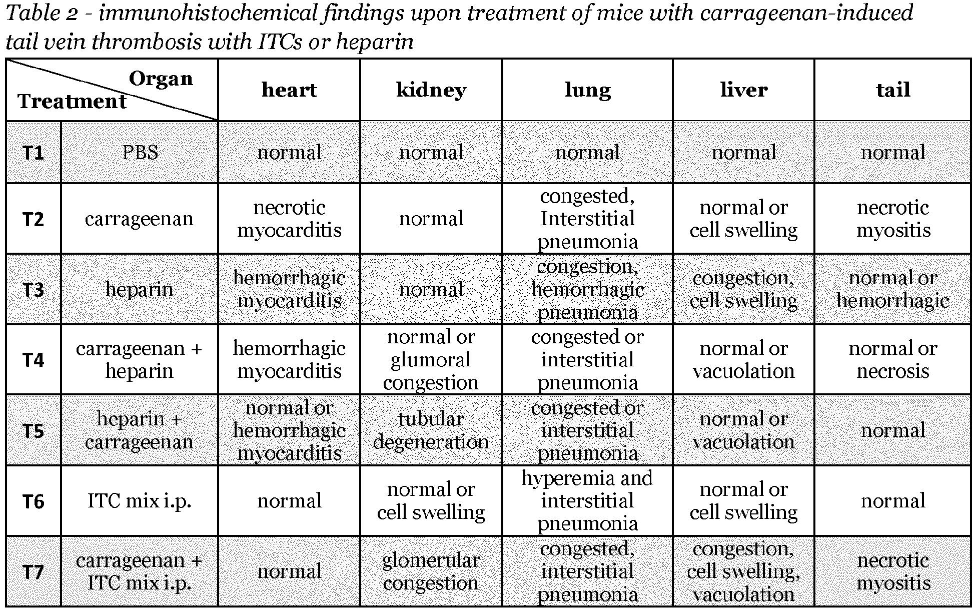

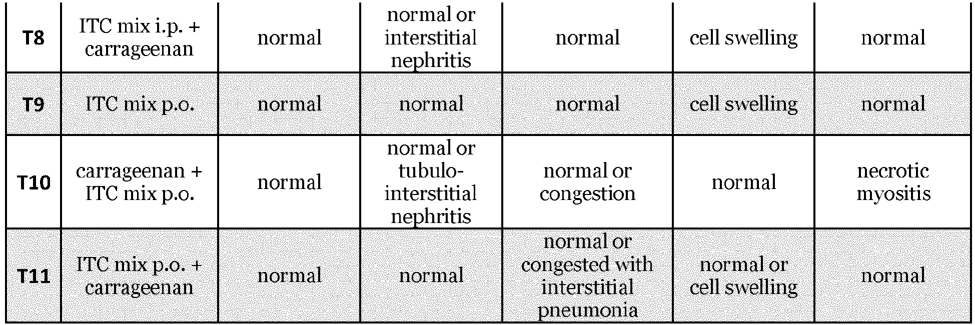

Taken together, all carrageenan-treated mice suffered from intravascular coagulation and thrombosis, as well as necrosis of the myocardium and tail muscles. Most symptoms were reversible after applying the therapeutic dose of the ITC mix i.p. or orally before or after thrombosis induction.

### Example 7

All methods mentioned in this example were carried out as described in Example 1.

### ITCs present and revert thrombosis in vivo

Finally, the efficacy of the ITC mix to prevent tail vein thrombosis and its effect on bleeding times were measured. Similar to heparin-treated mice, tail vein thrombosis was reduced in mice treated with ITCs before or after induction of tail vein thrombosis by carrageenan **(****Figure 13A**). Furthermore, the notion of anticoagulant properties of the ITC mix were supported by enhanced bleeding times and ODs, which was comparable to heparin-treated mice **(****Figure 13B** and **C**).

In summary, these data demonstrate that ITCs are effective in preventing and reversing thrombotic incidents in vivo.

### Example 8

### Conclusion

Regulation of PLT activation and aggregation is a complex and highly important physiological process, the deregulation is potentially lethal. The above examples show that a mixture of AITC, BITC and PEITC prevented PLT activation and aggregation and induced mitochondrial damage. Moreover, the ITCs also reversed PLT activation and aggregation, which provides new therapeutic uses for medicines containing ITCs. Reduced PLT activation and aggregation was paralleled by mitochondrial damage along with reduced GSH/GSSG ratios and enhanced PSSG levels **(****Figure 14**). Finally, the experiments show that the ITCs also prevented and reduced thrombosis in a carrageenan-induced murine thrombosis model.

The anti-aggregatory effect of ITCs on PLTs seems to be dose-dependent.

The single ITCs PEITC, BITC and AITC surprisingly showed strong effects on PLT aggregation (cf. **Figure 2**). Though the concentration of PEITC was the lowest among the ITCs, it had the strongest effect on PLT aggregation out of the three ITCs tested, whereas AITC had the weakest effect on PLT aggregation. Interestingly, the combination of two or three ITCs further increased the effect, arguing either for an additive effect or a dose-dependent effect of the different ITCs (or both).

For the in vitro experiments, 845 µM ITC mix were used, which corresponds to 111 mg per kg bodyweight assuming 100% bioavailability, which should be within the physiological range of ITCs in the body upon treatment with phytotherapeutics.

The ability to prevent PLT aggregation has been described for several compounds, such as GPIIb/IIIa antagonists (e.g., abciximab or tirofiban), COX inhibitors (e.g., aspirin or other non-steroidal anti-inflammatory drugs) or P2Y₁₂ antagonists (e.g., clopidogrel). GPIIb/IIIa, P2Y₁₂ or phosphatidylinositol-3 kinase antagonists were also effective to reverse ADP-induced PLT aggregation in vitro. However, while tirofiban prevented PLT aggregation *in vitro,* it further enhanced PLT activation markers and degranulation when PLTs were subjected to an activating stimulus. In contrast, the ITC mix or the single ITCs investigated in this work successfully prevented all: PLT activation, degranulation and aggregation in vitro. The above examples further demonstrate that when treated with the ITC mix after activation, aggregation of PLT was reduced and could even be reduced upon ITC application.

Inhibition of the AKT pathway is known to reduce PLT activation and aggregation, since it is crucial during PLT activation. In the present invention, dampened activation of this signaling pathway upon ITC treatment in PLTs was observed. Furthermore, the PamGene kinase assay suggested several other pathways involved in PLT activation to be diminished, such as PKC or Syk signaling. This indicates that these pathways may be a main mechanism by which PLT activation is prevented by ITCs. Moreover, ITC mix treatments reduced carrageenan-induced pulmonary thromboembolism, as well as tail and cardiac necrosis. In the experiments of the present application 1.3-2 mg/kg ITC mix was used, indicating that this mixture of ITCs is highly potent.

Taken together, the examples of the present application surprisingly and convincingly show that PEITC and BITC, the combination of PEITC and BITC together or each in combination with other ITCs, such as AITC, or the combination of the three ITCs can prevent PLT degranulation, activation and aggregation and reverse PLT aggregation and activation in vitro and in vivo.

Thrombotic disorders are one of the leading causes of death worldwide and their incidence has been increasing over the last decades. Therefore, prevention of PLT activation, degranulation and aggregation as well as reversion of PLT aggregation is highly important. The ability of the ITCs PEITC and BITC alone or in combination with each other and/or other ITCs, such as AITC, to prevent and resolve PLT aggregation in vivo and in vitro highlights their suitability for the novel therapeutic approach for the treatment and/or prevention of a thrombotic disorder including antiplatelet therapy and even thrombolysis.

## Claims

1. A composition comprising isothiocyanate for use in a method of treatment and/or prevention of a thrombotic disorder, wherein the isothiocyanate is selected from 2-phenylethyl-isothiocyanate (PEITC), benzyl-isothiocyanate (BITC), and/or combinations of PEITC and BITC, wherein, optionally, the composition further comprises one or more further isothiocyanate(s).

2. The composition for use according to claim 1, wherein the one or more further isothiocyanate(s) is (are) selected from a group comprising allyl-isothiocyanate (AITC), sulforaphane, and isopropyl isothiocyanate; wherein, preferably, the one or more further isothiocyanate(s) is (are) AITC.

3. The composition for use according to claim 1 or 2, wherein the composition for use comprises as isothiocyanate:
PEITC;
BITC;
a combination of PEITC and BITC;
a combination of PEITC and AITC;
a combination of BITC and AITC; or
a combination of PEITC, BITC, and AITC;
wherein, preferably, the composition for use comprises:
PEITC;
a combination of PEITC and BITC;
a combination of PEITC and AITC;
a combination of BITC and AITC; or
a combination of PEITC, BITC and AITC;
wherein, more preferably, the composition for use comprises:
a combination of PEITC and BITC;
a combination of PEITC and AITC; or
a combination of PEITC, BITC and AITC;
wherein, even more preferably, the composition for use comprises a combination of PEITC, BITC and AITC.

4. The composition for use according to any of the foregoing claims, wherein if the composition for use comprises a combination of PEITC, BITC and AITC, the mass ratio of PEITC : AITC : BITC is in a range from about 100 : 10 : 1 to about 1 : 10 : 100, preferably in a range from about 50 : 5 : 1 to about 1 : 5 : 50, even more preferably in a range from about 10 : 5 : 1 to about 1 : 5 : 10, most preferably the mass ratio is about 1 : 3.1 : 4.1; or
wherein if the composition for use comprises a combination of PEITC and BITC, the mass ratio of PEITC : BITC is in a range from about 1 : 50 to about 50 : 1, preferably in a range from about 1 : 10 to about 10 : 1, even more preferably in a range from about 1 : 5 to about 5 : 1; or
wherein if the composition for use comprises a combination of PEITC and AITC, the mass ratio of PEITC : AITC is in a range from about 1 : 50 to about 50 : 1, preferably in a range from about 1 : 10 to about 10 : 1, even more preferably in a range from about 1 : 5 to about 5 : 1; or
wherein if the composition for use comprises a combination of BITC and AITC, the mass ratio of BITC : AITC is in a range from about 1 : 50 to about 50 : 1, preferably in a range from about 1 : 10 to about 10 : 1, even more preferably in a range from about 1 : 5 to about 5 : 1.

5. The composition for use according to any of the foregoing claims, wherein the thrombotic disorder is caused by one or more thrombi and/or one or more emboli; and/or
wherein a cause of the thrombotic disorder includes an activation and/or degranulation and/or aggregation of platelets.

6. The composition for use according to any of the foregoing claims, wherein the thrombotic disorder is selected from a group comprising venous thrombosis or arterial thrombosis,
wherein, preferably, the venous thrombosis is selected from a group comprising deep vein thrombosis, portal vein thrombosis, renal vein thrombosis, jugular vein thrombosis, Budd-Chiari syndrome, Paget-Schroetter disease, cerebral venous sinus thrombosis, and mesenterial venous thrombosis; and
wherein, preferably, the arterial thrombosis is selected from a group comprising thrombotic stroke, myocardial infarction, pulmonary embolism, mesenterial infarction and acute arterial limb occlusion; and/or
wherein the thrombotic disorder affects an organ, preferably an organ selected from a group comprising the brain, the heart, the lung, the mesenterium, the kidney, and the liver.

7. The composition for use according to any of the foregoing claims, wherein the composition for use is capable of preventing platelet activation, platelet degranulation and/or platelet aggregation and/or wherein the composition for use is capable of reversing platelet aggregation and/or platelet activation; and/or
wherein the composition for use is capable of acting as an anti-coagulant, preferably as a platelet-active drug.

8. The composition for use according to any of the foregoing claims, wherein the composition for use is a pharmaceutical composition;
wherein, optionally, the pharmaceutical composition comprises one or more further drug(s) or agent(s), such as pharmaceutically acceptable excipient(s) and/or carrier(s).

9. The composition for use according to any of the foregoing claims, wherein the composition for use is administered to a patient in need thereof;
wherein, preferably, the patient is a human or an animal, preferably a human.

10. The composition for use according to any of the foregoing claims, wherein the composition for use is administered via a route of administration selected from a group comprising:
a parenteral route of administration, such as intravenous administration, administration via a catheter, subcutaneous administration, intraperitoneal administration, intramuscular administration, intraarterial administration, nasal administration, intra-articular administration, intracardiac administration, intradermal administration, intralesional administration, intraocular administration, intraosseous administration, intrathecal administration, intravaginal administration, intracavernous administration, intravesical administration, intravitreal administration, transdermal administration, transmucosal administration, and perivascular administration;
an enteral route of administration, such as oral administration, sublingual administration, buccal administration, and rectal administration;
wherein, preferably, the composition for use is administered via a route of administration selected from a group comprising intravenous administration, administration via a catheter, subcutaneous administration, intraperitoneal administration, and oral administration;
more preferably selected from intravenous administration, administration via a catheter, subcutaneous administration, and intraperitoneal administration;
even more preferably selected from intravenous administration, administration via a catheter, and subcutaneous administration;
most preferably the composition for use is administered via intravenous administration.

11. The composition for use according to any of the foregoing claims, wherein the composition for use is administered systematically or locally;
wherein, preferably, systemic administration is performed via intravenous administration and/or local administration is performed via a catheter.

12. The composition for use according to any of the foregoing claims, wherein the composition for use is administered in conjunction with further agent(s) or drug(s), such as one or more further anticoagulant(s), and/or
wherein the composition for use is administered in conjunction with mechanical thrombus fragmentation and/or aspiration thrombectomy (mechanical thrombectomy) and/or rheolytic thrombectomy.

13. The composition for use according to any of the foregoing claims, wherein the composition for use is formulated and administered as a solution, an injection, a pill, a tablet, a capsule, a suppository, an oral solution, a buccal, a granule, a powder, a suspension, a spray, an ointment, drops, or patches.

14. A portable medicine injection device comprising a composition for use as defined in any of the foregoing claims;
wherein, preferably, the medicine injection device is a pen-type drug delivery device;
more preferably, a disposable pen-type drug delivery device.

15. A kit comprising:
a first container comprising the composition for use according to any of claims 1-13;
optionally, a second container comprising a composition comprising a pharmaceutically acceptable diluent; and
optionally, means for the intravenous administration, subcutaneous administration, intraperitoneal administration and/or intramuscular administration of said composition for use, such as syringes;
wherein the container is selected from a group comprising vials, bottles, pre-filled syringes, and ampoules;
wherein, preferably, the composition for use is provided in a form selected from: an injection concentrate, a pre-diluted solution, and a lyophilized powder for reconstitution; more preferably an injection concentrate.
